# EUROPEAN PATENT APPLICATION

(11) **EP 3 272 771 A1**
(43) Date of publication of application: **24.01.2018**
(21) Application number: 16305943.9
(22) Date of filing: 22.07.2016
(51) Int. Cl.: C07K 16/22, A61P 35/00, A61K 31/00, A61K 39/395

(54) **TREATMENT FOR USE FOR PREVENTING METASTASIS IN A SUBJECT EXPOSED TO CANCER TREATMENT INDUCING P38 ACTIVATION**

(71) Applicant: CENTRE NATIONAL DE LA RECHERCHE SCIENTIFIQUE, 75794 Paris Cedex 16 (FR); Université Nice Sophia Antipolis, 06103 Nice Cedex 2 (FR); INSERM (Institut National de la Santé et de la Recherche Médicale), 75013 Paris (FR); Centre Scientifique de Monaco, 98000 Monaco (MC)
(72) Inventor: PAGES, Gilles, 98000 Monaco (MC); GREPIN, Renaud, 06240 Beausoleil (FR); DUFIES, Maeva Paola, 06200 Nice (FR)
(74) Representative: Cabinet Becker et Associés

(57) **Abstract**

The present disclosure relates to an anti-VEGFC antibody for use for preventing the occurrence of new metastases or the development of existing metastases in a subject exposed to or who will be exposed to a cancer treatment inducing p38 activation. The present disclosure in addition provides methods for selecting a treatment of interest, as well as pharmaceutical compositions, kits, and uses thereof.

SEQUENCE LISTING

<110>  CENTRE NATIONAL DE LA RECHERCHE SCIENTIFIQUE et al.

<120>  Treatment for use for preventing metastasis in a subject exposed
       to cancer treatment inducing p38 activation

<130>  B2282EP00

<160>  28

<170>  PatentIn version 3.5

<210>  1
<211>  36
<212>  DNA
<213>  Artificial Sequence

<220>
<223>  3' UTR with VEGFC ARE

<400>  1
gatttcttta aaagaatgac tatataattt atttcc                                36


<210>  2
<211>  36
<212>  DNA
<213>  Artificial Sequence

<220>
<223>  3'UTR with Mutated ARE

<400>  2
gatttcttta aaagaatgac tatataatct atttcc                                36


<210>  3
<211>  20
<212>  DNA
<213>  Artificial Sequence

<220>
<223>  Forward primer

<400>  3
agaaggagga gggcagaatc                                                  20


<210>  4
<211>  20
<212>  DNA
<213>  Artificial Sequence

<220>
<223>  Reverse primer

<400>  4
tctcgattgg atggcagtag                                                  20


<210>  5
<211>  26

<212>  DNA
<213>  Artificial Sequence

<220>
<223>  Taqman probe

<400>  5
catccatgaa cttcaccact tcgtga                                          26


<210>  6
<211>  20
<212>  DNA
<213>  Artificial Sequence

<220>
<223>  Forward primer

<400>  6
ggatgctgga gatgactcaa                                                 20


<210>  7
<211>  20
<212>  DNA
<213>  Artificial Sequence

<220>
<223>  Reverse primer

<400>  7
ttcatccagc tccttgtttg                                                 20


<210>  8
<211>  26
<212>  DNA
<213>  Artificial Sequence

<220>
<223>  Taqman probe

<400>  8
tccacagatg tcatggaatc catctg                                          26


<210>  9
<211>  448
<212>  PRT
<213>  Artificial Sequence

<220>
<223>  Anti-VEGFC antibody (VGX-100) heavy chain

<400>  9

Glu Val Arg Leu Leu Glu Ser Gly Gly Gly Leu Val Gln Pro Gly Gly
1               5                   10                  15


Ser Leu Arg Leu Ser Cys Ala Ala Ser Gly Phe Thr Phe Arg Pro Arg
            20                  25                  30

Ala Met Ala Trp Val Arg Gln Ala Pro Gly Lys Gly Leu Glu Trp Val
        35                  40                  45

Ser Ser Ile Ser Ala Gln Gly Ala Ser Ala Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Ser Val Ser Gly Phe Gly Pro Trp Gly Arg Gly Thr
            100                 105                 110

Met Val Thr Val Ser Ser Ala Ser Thr Lys Gly Pro Ser Val Phe Pro
        115                 120                 125

Leu Ala Pro Ser Ser Lys Ser Thr Ser Gly Gly Thr Ala Ala Leu Gly
    130                 135                 140

Cys Leu Val Lys Asp Tyr Phe Pro Glu Pro Val Thr Val Ser Trp Met
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Leu Gly Gly Pro Ser
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Asn Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Glu Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Val Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro Gly Lys
        435                 440                 445

<210>  10
<211>  217
<212>  PRT
<213>  Artificial Sequence

<220>
<223>  Anti-VEGFC antibody (VGX-100) light chain

<400>  10

Ser Tyr Glu Leu Thr Gln Pro Pro Ser Ser Ser Gly Thr Pro Gly Gln
1               5                   10                  15

Arg Val Thr Ile Ser Cys Ser Gly Ser Ser Ser Asn Ile Gly Arg His
            20                  25                  30

Thr Val Ser Trp Tyr Gln Gln Val Pro Gly Thr Ala Pro Lys Leu Leu
        35                  40                  45

Ile Tyr Ser Asp Asp His Arg Pro Ser Gly Val Pro Asp Arg Phe Ser
    50                  55                  60

Ala Ser Lys Ser Gly Thr Ser Ala Ser Leu Thr Ile Thr Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Trp Val Phe Gly Gly Gly Thr Lys Leu Thr Val Leu Gly
            100                 105                 110

Gln Pro Lys Ala Ala Pro Ser Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Ser Ser Pro Val
145                 150                 155                 160

Lys Ala Gly Val Glu Thr Thr Thr Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215

<210>  11
<211>  118
<212>  PRT
<213>  Artificial Sequence

<220>
<223>  Anti-VEGFC antibody (VGX-100) heavy chain variable region (VH)

<400>  11

Glu Val Arg Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Pro Arg
            20                  25                  30

Ala Met Ala Trp Val Arg Gln Ala Pro Gly Lys Gly Leu Glu Trp Val
        35                  40                  45

Ser Ser Ile Ser Ala Gln Gly Ala Ser Ala Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Leu Ser Val Ser Gly Phe Gly Pro Trp Gly Arg Gly Thr
            100                 105                 110

Met Val Thr Val Ser Ser
        115

<210>  12
<211>  112
<212>  PRT
<213>  Artificial Sequence

<220>
<223>  Anti-VEGFC antibody (VGX-100) light chain variable region (VL)

<400>  12

Ser Tyr Glu Leu Thr Gln Pro Pro Ser Ser Ser Gly Thr Pro Gly Gln
1               5                   10                  15

Arg Val Thr Ile Ser Cys Ser Gly Ser Ser Ser Asn Ile Gly Arg His
            20                  25                  30

Thr Val Ser Trp Tyr Gln Gln Val Pro Gly Thr Ala Pro Lys Leu Leu
        35                  40                  45

Ile Tyr Ser Asp Asp His Arg Pro Ser Gly Val Pro Asp Arg Phe Ser
    50                  55                  60

Ala Ser Lys Ser Gly Thr Ser Ala Ser Leu Thr Ile Thr Gly Leu Gln
65                  70                  75                  80

Ser Glu Asp Glu Ala Asp Tyr Tyr Cys Ala Ala Trp Asp Asp Ser Leu
                85                  90                  95

Asn Gly Pro Trp Val Phe Gly Gly Gly Thr Lys Leu Thr Val Leu Gly
            100                 105                 110

<210>  13

<211>  122
<212>  PRT
<213>  Artificial Sequence

<220>
<223>  Anti-VEGFC antibody (VC4.5) heavy chain

<400>  13

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Thr Asp Asn
            20                  25                  30

Trp Ile His Trp Val Arg Gln Ala Pro Gly Lys Gly Leu Glu Trp Val
        35                  40                  45

Gly Val Ile Ser Pro Tyr Ser Gly Ala Thr Tyr Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Asp Ala Asp Tyr Lys Tyr Ala Phe Trp Ala Phe Asp Tyr Trp
            100                 105                 110

Gly Gln Gly Thr Leu Val Thr Val Ser Ser
        115                 120


<210>  14
<211>  108
<212>  PRT
<213>  Artificial Sequence

<220>
<223>  Anti-VEGFC antibody (VC4.5) light chain

<400>  14

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Thr Pro Pro
                85                  90                  95

Thr Phe Gly Gln Gly Thr Lys Val Glu Ile Lys Arg
            100                 105

<210>  15
<211>  11
<212>  PRT
<213>  Artificial Sequence

<220>
<223>  "VC4.5" CDR1

<400>  15

Arg Ala Ser Gln Asp Val Ser Thr Ala Val Ala
1               5                   10

<210>  16
<211>  7
<212>  PRT
<213>  Artificial Sequence

<220>
<223>  "VC4.5" CDR2

<400>  16

Ser Ala Ser Phe Leu Tyr Ser
1               5

<210>  17
<211>  9
<212>  PRT
<213>  Artificial Sequence

<220>
<223>  "VC4.5" CDR3

<400>  17

Gln Gln Ser Tyr Thr Thr Pro Pro Thr
1               5

<210>  18
<211>  10
<212>  PRT
<213>  Artificial Sequence

<220>
<223>  "VC4.5" CDR1

<400>  18

Gly Phe Thr Phe Thr Asp Asn Trp Ile His
1               5                   10


<210>  19
<211>  18
<212>  PRT
<213>  Artificial Sequence

<220>
<223>  "VC4.5" CDR2

<400>  19

Gly Val Ile Ser Pro Tyr Ser Gly Ala Thr Tyr Tyr Ala Asp Ser Val
1               5                   10                  15


Lys Gly


<210>  20
<211>  12
<212>  PRT
<213>  Artificial Sequence

<220>
<223>  "VC4.5" CDR3

<400>  20

Ala Arg Asp Ala Asp Tyr Lys Tyr Ala Phe Trp Ala
1               5                   10


<210>  21
<211>  117
<212>  PRT
<213>  Artificial Sequence

<220>
<223>  Anti-VEGFC antibody (VC1.12) heavy chain

<400>  21

Glu Val Gln Leu Val Glu Ser Gly Gly Gly Leu Val Gln Pro Gly Gly
1               5                   10                  15


Ser Leu Arg Leu Ser Cys Ala Ala Ser Gly Phe Thr Phe Ser Asp Ser
            20                  25                  30


Asp Ile His Trp Val Arg Gln Ala Pro Gly Lys Gly Leu Glu Trp Val
        35                  40                  45

Ala Trp Ile Ser Pro Tyr Val Gly Phe Thr Asp Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ala Arg Leu Phe Asp Val Ile Phe Asp Tyr Trp Gly Gln Gly Thr Leu
            100                 105                 110

Val Thr Val Ser Ser
        115

<210>  22
<211>  108
<212>  PRT
<213>  Artificial Sequence

<220>
<223>  Anti-VEGFC antibody (VC1.12) light chain

<400>  22

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Ser Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Thr Thr Pro Pro
                85                  90                  95

Thr Phe Gly Gln Gly Thr Lys Val Glu Ile Lys Arg
            100                 105

<210>  23
<211>  11
<212>  PRT
<213>  Artificial Sequence

<220>
<223>  "VC1.12" CDR1

<400>  23

Arg Ala Ser Gln Asp Val Ser Thr Ala Val Ala
1               5                   10


<210>  24
<211>  7
<212>  PRT
<213>  Artificial Sequence

<220>
<223>  "VC1.12" CDR2

<400>  24

Ser Ala Ser Phe Leu Tyr Ser
1               5


<210>  25
<211>  9
<212>  PRT
<213>  Artificial Sequence

<220>
<223>  "VC1.12" CDR3

<400>  25

Gln Gln Ser Tyr Thr Thr Pro Pro Thr
1               5


<210>  26
<211>  10
<212>  PRT
<213>  Artificial Sequence

<220>
<223>  "VC1.12" CDR1

<400>  26

Gly Phe Thr Phe Ser Asp Ser Asp Ile His
1               5                   10


<210>  27
<211>  18
<212>  PRT
<213>  Artificial Sequence

<220>
<223>  "VC1.12" CDR2

<400>  27

Ala Trp Ile Ser Pro Tyr Val Gly Phe Thr Asp Tyr Ala Asp Ser Val
1               5                   10                  15

Lys Gly

<210> 28
<211> 10
<212> PRT
<213> Artificial Sequence

<220>
<223> "VC1.12" CDR3

<400> 28

Ala Arg Leu Phe Asp Val Ile Phe Asp Tyr
1               5                   10

## Description

### FIELD OF THE INVENTION

The present disclosure generally relates to the field of medicine. The present invention more specifically relates to an anti-VEGFC antibody, or a functional fragment thereof, for use for preventing the occurrence of new metastases or the development of existing metastases in a subject exposed to or who will be exposed to a cancer treatment inducing p38 activation.

Inventors in particular herein identify for the first time axitinib, pazopanib, imatinib, taxans and platinum salts as cancer treatments inducing p38 activation.

The present invention in addition provides methods for selecting a treatment of interest, as well as pharmaceutical compositions, kits, and uses thereof.

### BACKGROUND OF THE INVENTION

Renal cell carcinoma (RCC) represents 85% of kidney cancers and 3% of adult cancers. However, its incidence has steadily increased. If diagnosed early, the main treatment is surgery. However, metastatic RCC has a very poor prognosis because of intrinsic resistance to radio- and chemo-therapy. The main feature of RCC is hyper-vascularization explained by overexpression of vascular endothelial growth factor (VEGF), which is linked to mutation/inactivation of the von Hippel-Lindau (*vhl*) gene - an E3 ubiquitin ligase of the Hypoxia Inducible-Factor 1α (HIF-1α). The most widely used systemic therapy for first-line metastatic RCC is sunitinib, a tyrosine kinase inhibitor (TKI) with activity against the VEGF receptors (VEGFR1/2/3), PDGFR, CSF1R and c-Kit (Eisen*et al,* 2012). Axitinib and pazopanib can also be used as a systemic therapy against metastatic RCC. However, treatment benefits are transitory in most cases and the majority of patients develop resistance after one year (Escudier, 2010). While the primary target of sunitinib is the host blood vessels, mechanisms of resistance have been shown to stem from complex interactions between tumor and stromal cell populations (Giuliano & Pages, 2013). Indeed, several mechanisms have been proposed and can include compensatory growth factor stimulation (i.e., FGF), suppression of immune-regulatory cells, or even co-option of existing blood vessels, all of which together (or separately) could negate the impact of anti-vascular treatment strategies (Giuliano & Pages, 2013; Ribatti, 2016; Wang*et al,* 2015; Welti*et al,* 2013). Perhaps most provocatively, recent preclinical evidence has shown that anti-angiogenic treatment may elicit metastatic cell phenotypes which, in turn, may also compromise tumor-reducing benefits (Ebos*et al,* 2009; Paez-Ribes*et al,* 2009). Currently more than 25 preclinical studies have confirmed this phenomena (Ebos, 2015), however, the clinical impact is not known. Antiangiogenic therapy-induced metastasis may explain disease progression following perioperative (adjuvant) treatment or rebound tumor growth after treatment withdrawal (Blagoev*et al,* 2013; Miles*et al,* 2011).

During tumor development, the lymphatic system is considered one of the primary routes of tumor cell dissemination that leads to distant metastatic growth. VEGFC is currently the best characterized lymphangiogenic factor that acts *via* VEGFR3 (Alitalo & Detmar, 2012). In normal adult tissues, VEGFR3 expression is largely restricted to lymphatic endothelial cells (LECs) and its activation is responsible for LEC proliferation, migration and survival. Several reports indicate that VEGFC expression in cancer cells correlates with accelerated tumor progression and/or an unfavorable clinical outcome (Su*et al,* 2007). VEGFC overexpression in breast cancers has been shown to correlate with lymphangiogenesis and metastasis (Wang *et al,* 2012). In preclinical models of RCC, endothelial cells chronically exposed to an anti-VEGF antibody proliferate in response to VEGFC stimulation whereas naive endothelial cells are unable to do so (Grau*et al,* 2011). Moreover, in a preclinical model of lung cancer, resistance to Aflibercept (a decoy receptor for VEGF and P1GF) is related to an increase in VEGFC (Li*et al,* 2014). The VEGFC mRNA has a long 3' untranslated domain (3'UTR) containing an adenylate and uridylate-rich element (ARE). ARE elements are binding sites for the embryonic lethal abnormal vision (ELAV) protein, also named HuR (Hu antigen R), and tristetraprolin (TTP), also named ZFP36 (zinc finger protein 36). These mRNA-binding proteins have been described previously as mRNA stabilizing and destabilizing factors, respectively. HuR stabilizes the mRNA of cell cycle regulators, growth, inflammatory and angiogenesis factors including VEGF (Levy*et al,* 1998). These features give HuR an oncogenic status (Wang*et al*, 2013). TTP has an opposite role (destabilization of these ARE-mRNA including VEGF), hence acting as a potent tumor suppressor (Brennan*et al,* 2009). The extracellular signal-regulated kinases (ERK) and mitogen-activated protein kinase p38 phosphorylate HuR and TTP. While ERK and p38-dependent phosphorylation activate the mRNA stabilizing activity of HuR, phosphorylation has an opposite effect on TTP. The balance between TTP and HuR will determine mRNA stabilization or degradation (Griseri & Pages, 2014). Although VEGFC plays a causal role in lymphangiogenesis and lymphatic metastasis, little is known about VEGFC regulation in tumor cells in response to anti-angiogenic treatments.

Inventors herein reveal the molecular mechanism linking particular antiangiogenic factors, preferably sunitinib, axitinib, pazopanib and imatinib, and also taxans and platinum salts (also herein identified as "platin(s)") to lymphangiogenesis activation through p38 activation, and subsequently through stimulation of *vegfc* gene transcription and stabilization of VEGFC mRNA. Inventors in particular reveal for the first time that p38 activation and VEGFC up-regulation induced by axitinib, pazopanib, imatinib, taxans or platin treatment correlates with the development of a lymphatic network both in tumors in mice and in patients. Their findings demonstrate that benefits of cancer treatment resulting in p38 activation are compromised by stimulation of lymphatic vessel formation and explain compensatory pro-metastatic behaviors which may compromise treatment efficacy.

Other advantages of the methods, products and compositions herein described are further indicated below.

### SUMMARY OF THE INVENTION

Herein described is a treatment, in particular an anti-VEGFC antibody, or a functional fragment thereof, for use for preventing the occurrence of new metastases or the development of existing metastases in a subject exposed to or who will be exposed to a cancer treatment inducing p38 activation, typically selected from axitinib, pazopanib, imatinib, a taxan and a platin.

Also herein described is a cancer treatment inducing p38 activation, typically selected from axitinib, pazopanib, imatinib, a taxan and a platin, for use in combination with an anti-VEGFC antibody, or with a functional fragment thereof, for treating a cancer and preventing metastasis in a subject in need thereof, preferably for use sequentially, the anti-VEGFC antibody being typically administered after the cancer treatment inducing p38 activation.

The present description also provides a method for selecting an optimal therapeutic treatment of cancer in a subject having metastasis(es) exposed to a cancer treatment inducing p38 activation, typically selected from axitinib, pazopanib, imatinib, a taxan and a platin, wherein the method comprises a step of assessing p38 activation *in vitro* or *ex vivo* in at least one metastasis or metastatic cell of the subject, and a step of deciding that the cancer treatment inducing p38 activation is to be combined to the administration to the subject of an anti-VEGFC antibody, or a functional fragment thereof, as the optimal therapeutic treatment of cancer for the subject, if an activation of p38 is detected in the at least one metastasis or metastatic cell of the subject.

Herein described is also a method of preventing metastasis occurrence or development, or of treating cancer and preventing metastasis occurrence or development, in a subject in need thereof, comprising the administration to the subject of an anti-VEGFC antibody, or a functional fragment thereof, in addition to a cancer treatment inducing p38 activation (as a combined preparation) or following a first administration to the subject of such a cancer treatment inducing p38 activation.

The present description further provides a kit comprising (i) a cancer treatment inducing p38 activation, typically selected from axitinib, pazopanib, imatinib, a taxan and a platin, and (ii) an anti-VEGFC antibody, or a functional fragment thereof, and inventors herein describe the advantageous use of such a kit for treating cancer and preventing the occurrence of new metastasis(es) or the development of existing metastasis(es) in a subject.

Further herein described is a kit comprising at least one anti-p38 antibody, or a functional fragment thereof, for detecting p38 activation in at least one metastasis or metastatic cell from a subject suffering of a cancer, and at least one secondary antibody detecting the at least one anti-p38 antibody, optionally together with an anti-VEGFC antibody.

### LEGEND TO THE FIGURES

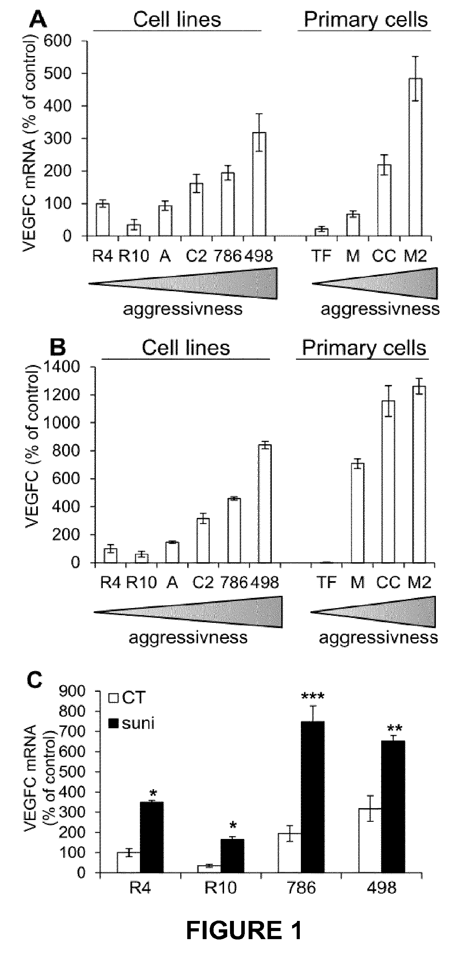

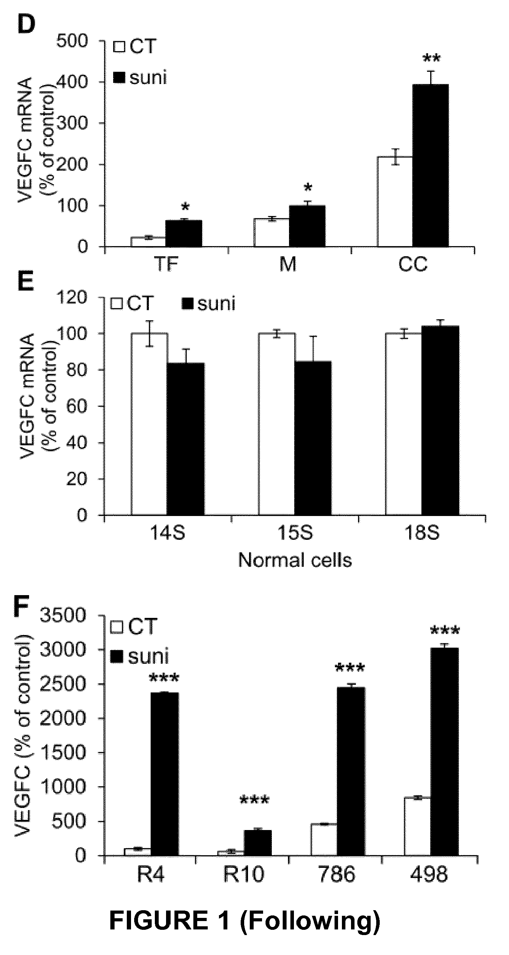

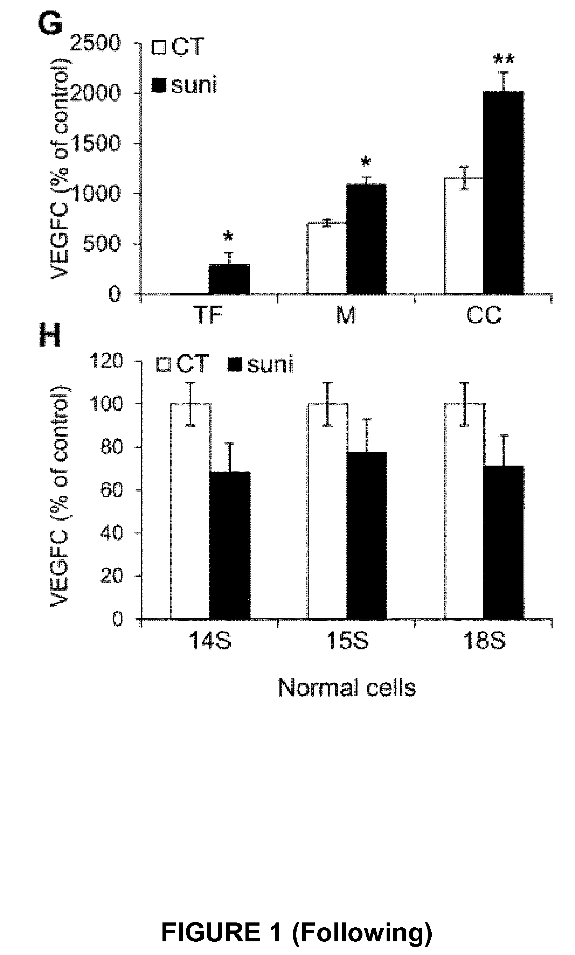

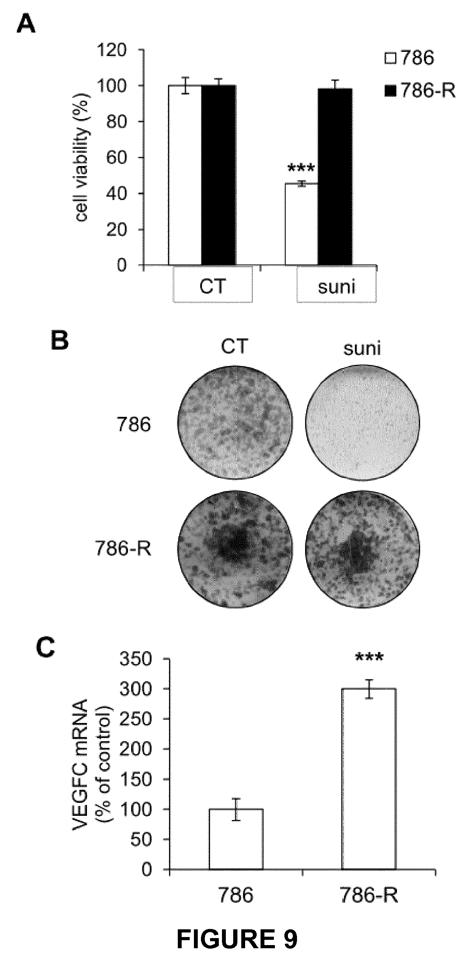

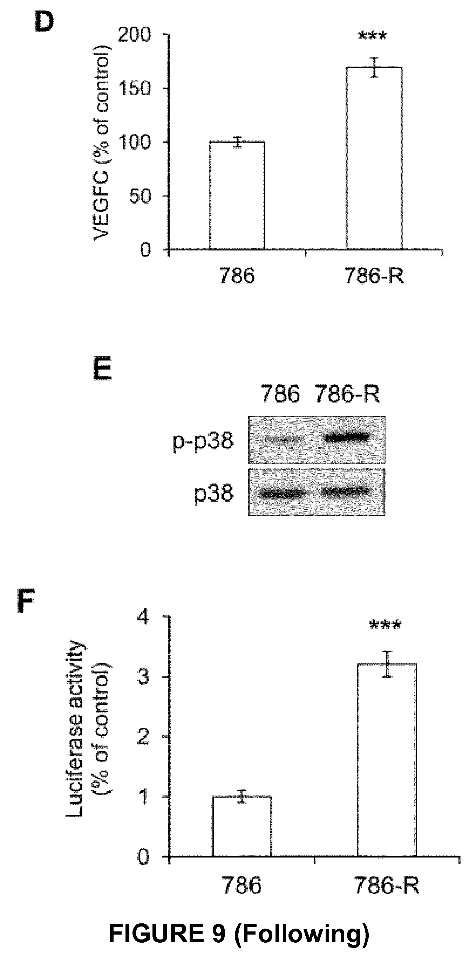

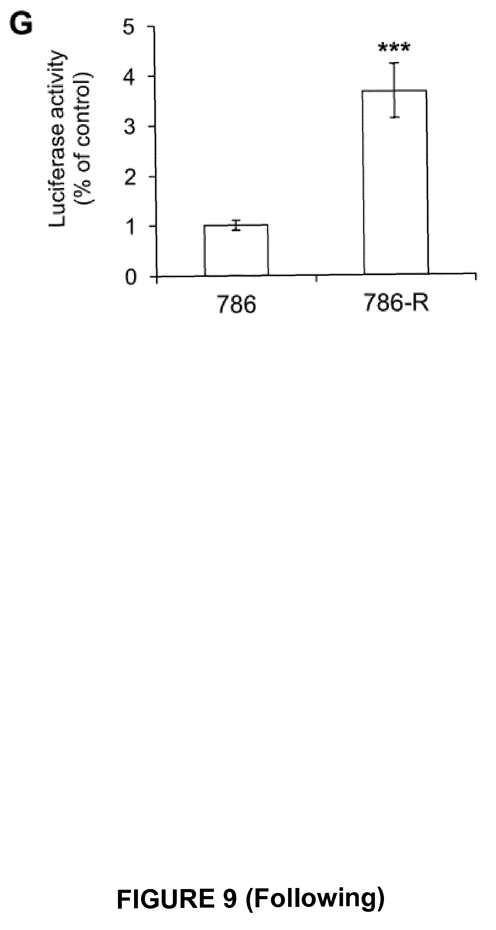

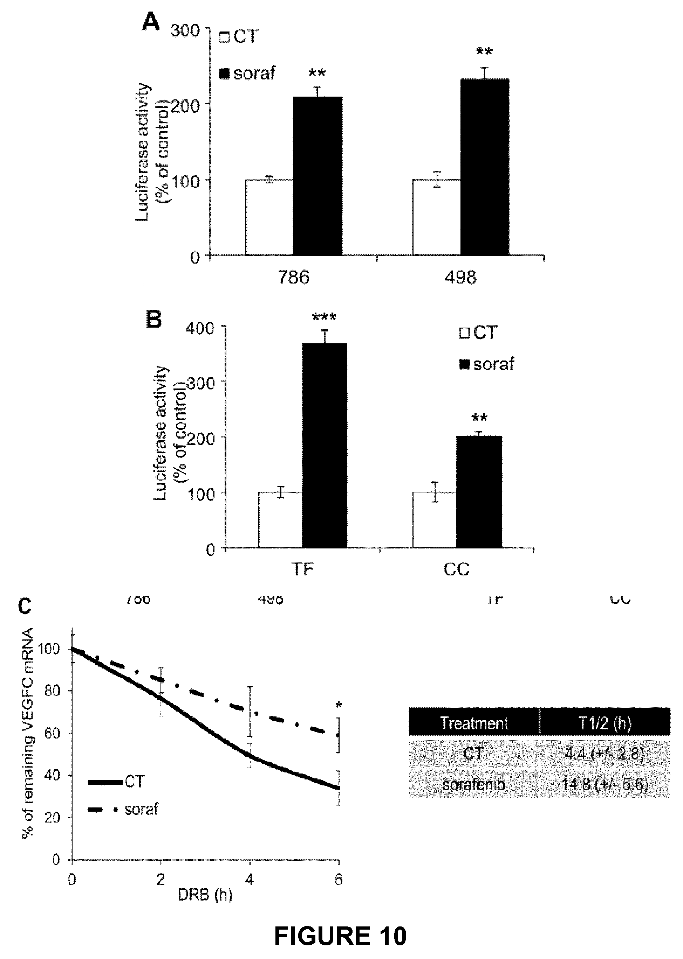

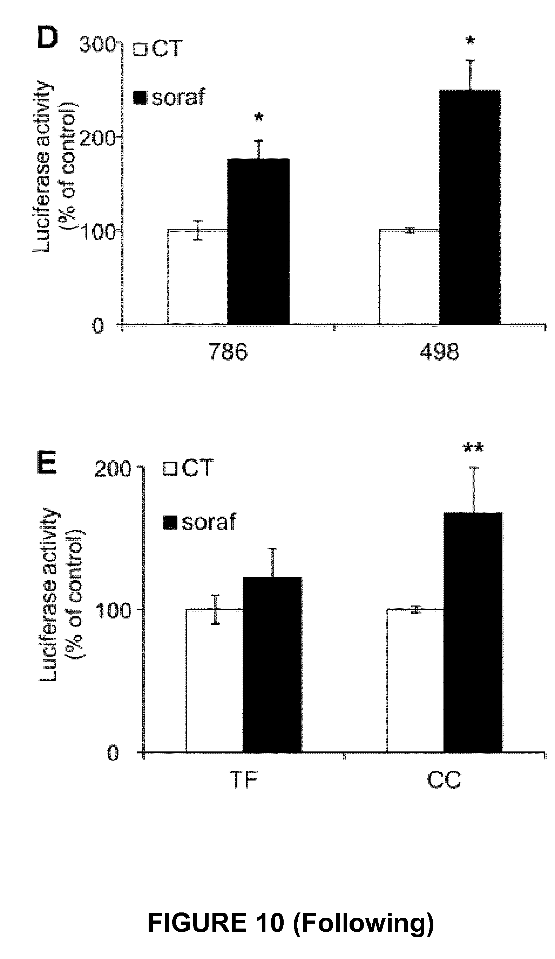

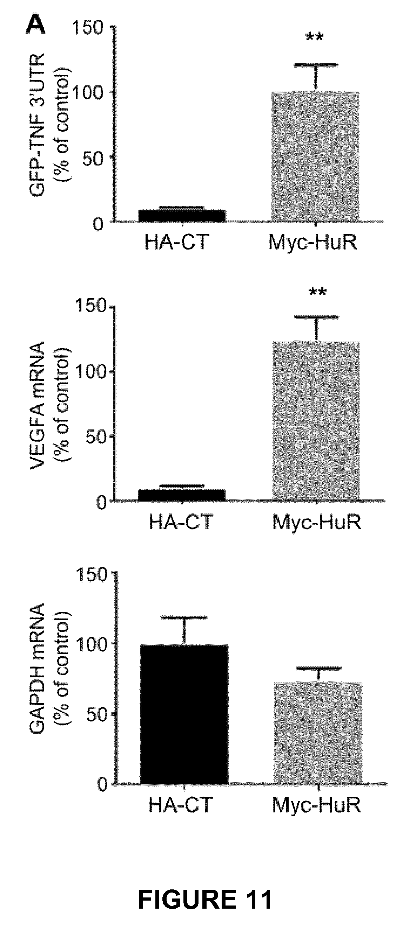

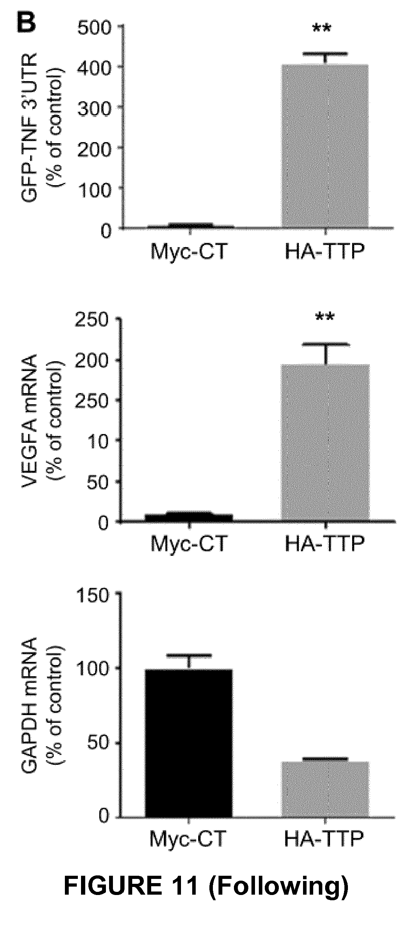

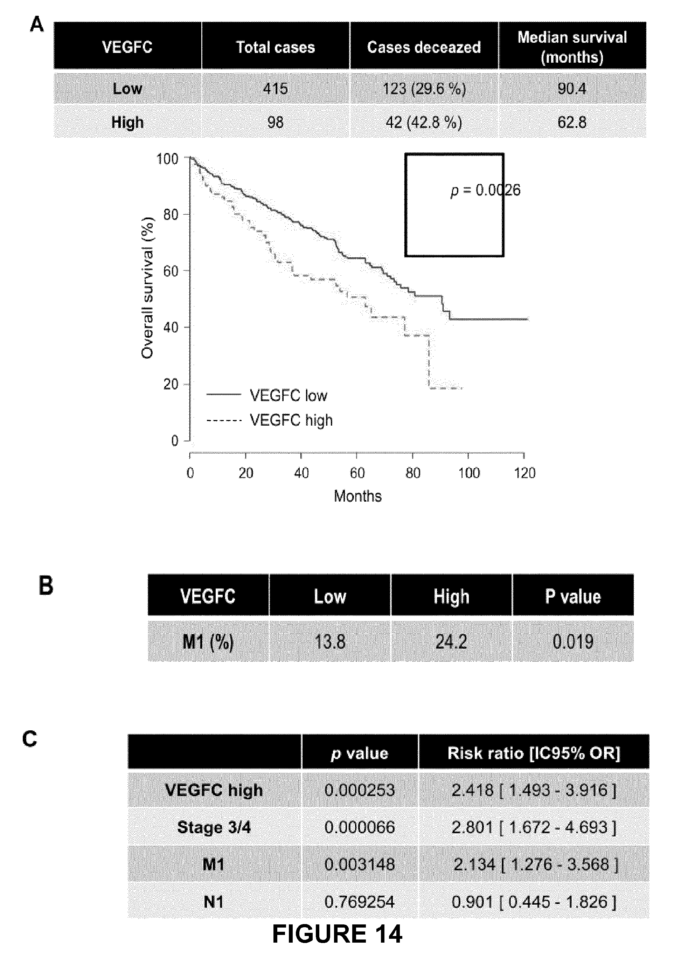

**Figure 1****: Sunitinib increased VEGFC expression.**
   Different RCC cell lines [(RCC4 (R4), RCC10 (R10), ACHN (**A**), Caki-2 (C2), 786-O (786) and A498 (498)] and primary RCC cells (TF, M, CC and M2) and normal renal cells (14S, 15S and 18S) were evaluated for VEGFC mRNA levels by qPCR (**A**, **C**, **D**, **E**) and for VEGFC protein in cell supernatants by ELISA (**B, F, G, H**). **C** and **F,** RCC cell lines were treated with 5 µM sunitinib (suni) for 48 hr. **D** and **G,** primary cells were treated with sunitinib (10 µM for TF, and 5 µM for M and CC) for 48 hr. **E** and **H,** normal kidney cells were treated with 5 µM sunitinib for 48 hr.
   For **A**, **C** and **D**, the mRNA level of R4 is considered as the reference value (100%). Data are represented as mean of 3 independent experiments ± SEM. * *p*<0.05, ** *p*<0.01, *** *p*<0.001.

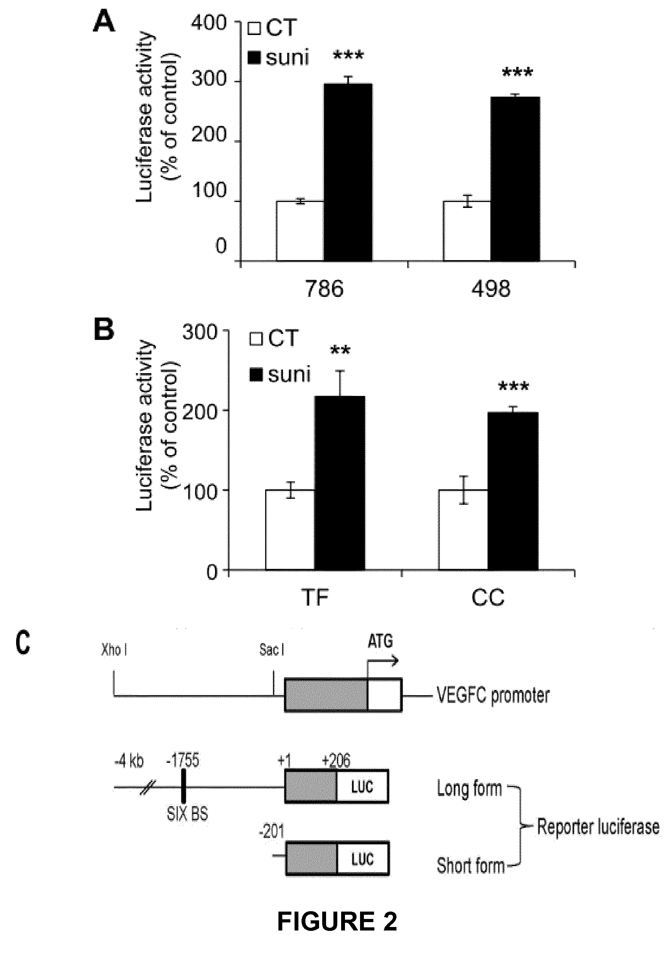

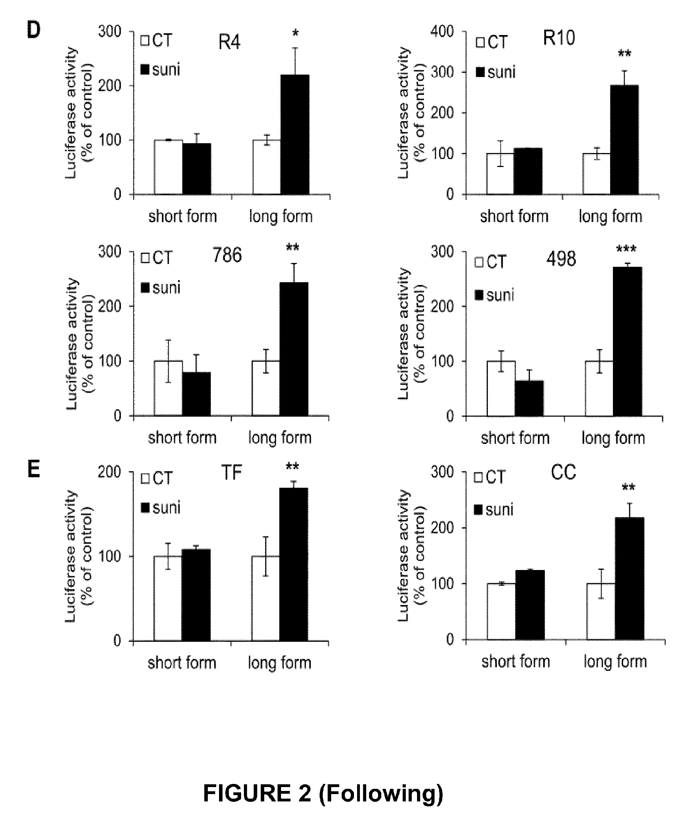

**Figure 2****: Sunitinib increased VEGFC promoter activity.**
   **A** and **B**, RCC cell lines (**A**) and primary cells (**B**) were transfected with a renilla luciferase reporter gene under the control of the VEGFC promoter and treated with sunitinib (suni) 2.5 µM for cell lines or 5 µM for primary cells for 24 hr. The renilla luciferase activity normalized to the firefly luciferase (control vector) was the readout of the VEGFC promoter activity. **C,** schemas of truncated short and long forms of VEGFC promoter activity reporter genes used in **D** and **E. D** and **E,** RCC cell lines (**D**) and primary cells (**E**) were transfected with a firefly luciferase reporter gene under the control of the truncated short or long form VEGFC promoter and treated with sunitinib 2.5 µM for cell lines or 5 µM for primary cells for 24 hr. Normalized luciferase activity (control vector) was the readout of the VEGFC promoter activity. Data are represented as mean of 3 independent experiments ± SEM. * p<0.05, ** p<0.01, *** p<0.001. See also Figure 9.

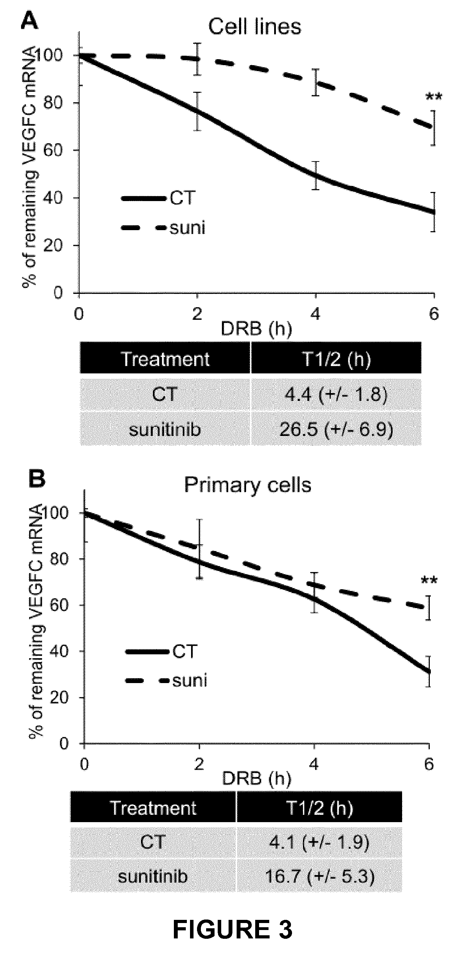

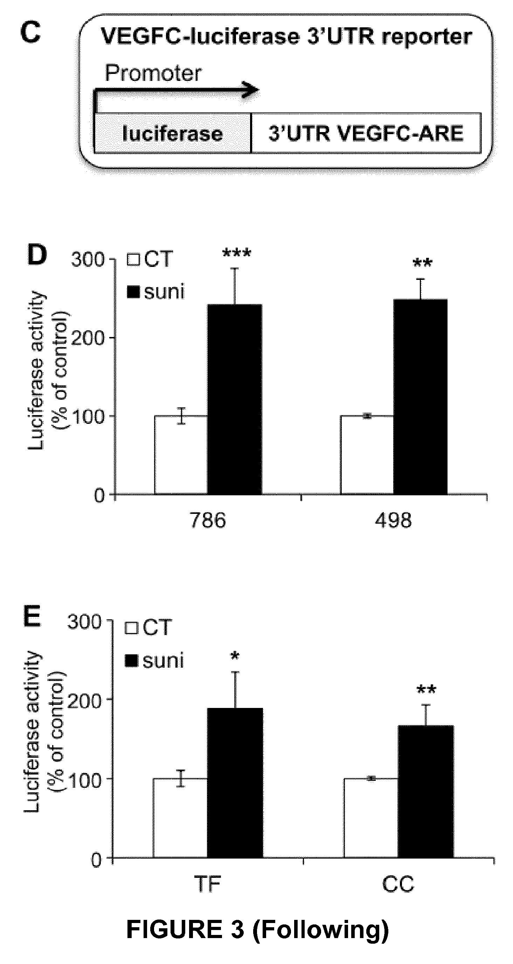

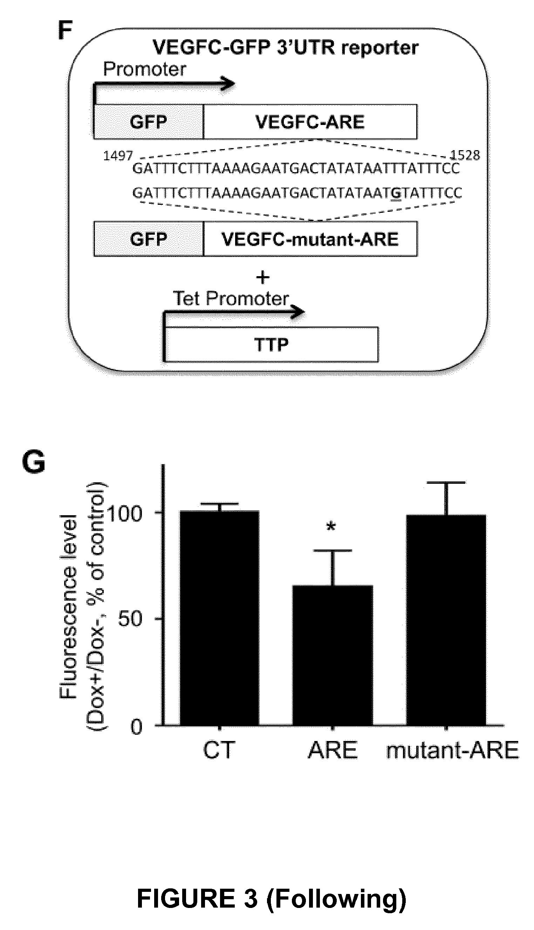

**Figure 3****: Sunitinib increased the half-life of VEGFC mRNA via its 3'UTR.**
   **A** and **B**, the RCC cell line (786-O) (**A**) or primary cells (CC) (**B**) were treated with 5 µM sunitinib (suni) for 48 hr. Cells were then incubated with DRB for 2, 4 or 6 hr. The remaining VEGFC mRNA was evaluated by qPCR and its mRNA **half-life** was calculated. C, Schema of the VEGFC luciferase 3'UTR reporter gene. **D** and **E,** RCC cell lines (**D**) or primary cells (**E**) were transfected with the VEGFC 3'UTR reporter gene and treated with sunitinib 5 µM (or 10 µM for TF) for 24 hr. The normalized luciferase activity was the readout of the reporter gene mRNA half-life. **F**, Schemas of VEGFC GFP 3'UTR wild-type or mutated for the ARE element reporter genes. **G**, cells were transfected with the VEGFC GFP 3'UTR wild-type or mutated reporter vectors for the VEGFC 3'UTR ARE. TTP expression was induced with 0.25 µg/mL doxycycline (dox). The fluorescence level was the readout of the reporter gene half-life. Data are represented as mean of 3 independent experiments ± SEM. * p<0.05, ** p<0.01, *** p<0.001. See also Figure 10.

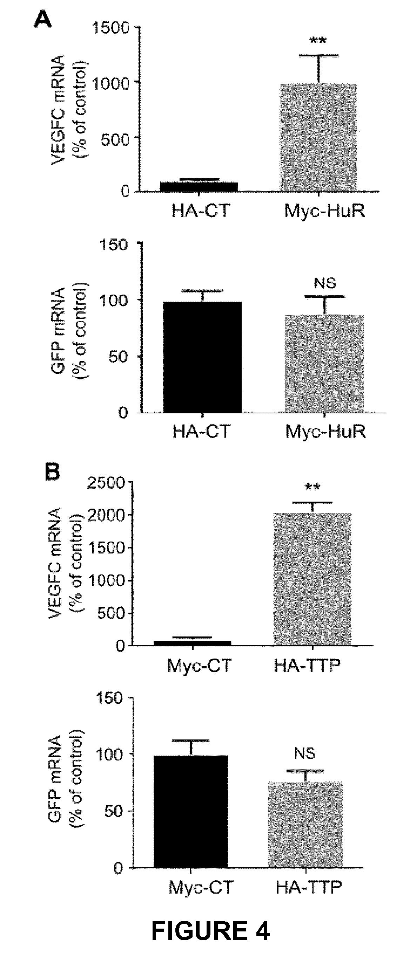

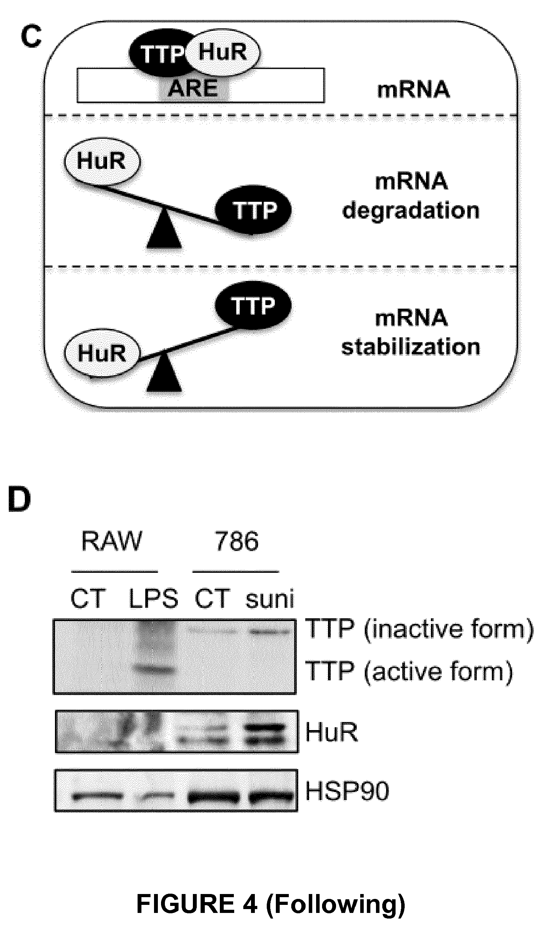

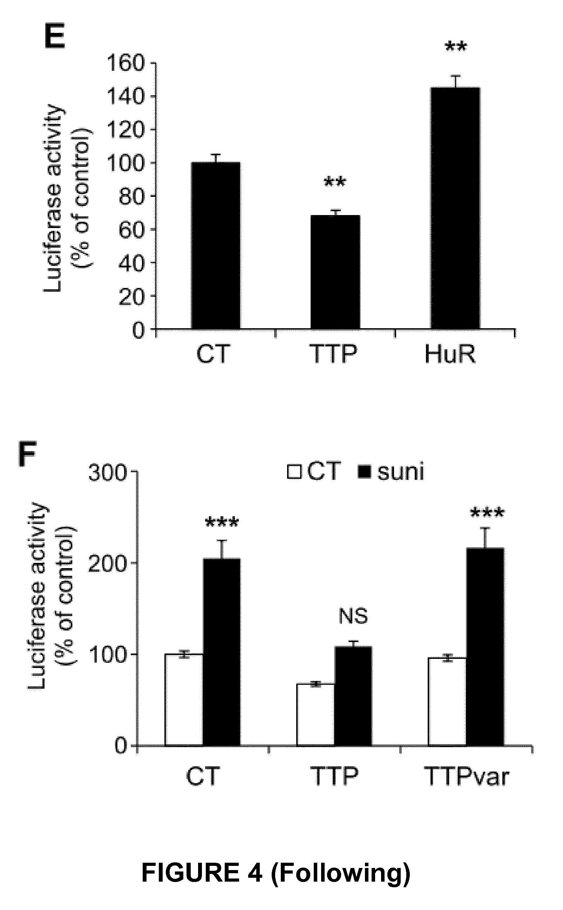

**Figure 4****: TTP and HuR bind VEGFC mRNA and modulate its half-life.**
   **A** and **B**, HuR/TTP interactions with VEGFC mRNA were analyzed by RIP-Chip (RNA-IP). HEK-293 cells were transfected with Myc-HuR or HA-CT (used as a negative control) (**A**) or with HA-TTP and Myc-CT (used as a negative control) (**B**). Exogenous TTP and HuR cross linked to mRNA were immunoprecipitated with anti-HA and anti-Myc antibodies. The levels of immunoprecipitated VEGFC or GFP mRNA (used as a negative control) were assessed by qPCR. **C**, schematic balance between TTP and HuR, and its effect on mRNA stability. **D**, RAW cells were stimulated with 10 µM lipopolysaccharide (LPS) for 6 hr and used as a positive control for active (unphosphorylated) forms of TTP. 786-Ocells were stimulated with 5 µM sunitinib (suni) for 6 hr. TTP and HuR expression was analyzed by western blot. HSP90 served as a loading control. **E** and **F,** 786-Ocells were transfected with a VEGFC luciferase 3'UTR reporter gene in the presence of expression vectors for HuR, TTP or TTPvar [a mutation that induces a decrease in TTP mRNA translation and serves as a negative control (Griseri *et al,* 2011)] and treated or not with sunitinib 5 µM for 24 hr. The normalized luciferase counts served as readout of the reporter gene mRNA half-life. Data are represented as mean of 3 independent experiments ± SEM. ** *p*<0.01, *** *p*<0.001. See Figure 11.

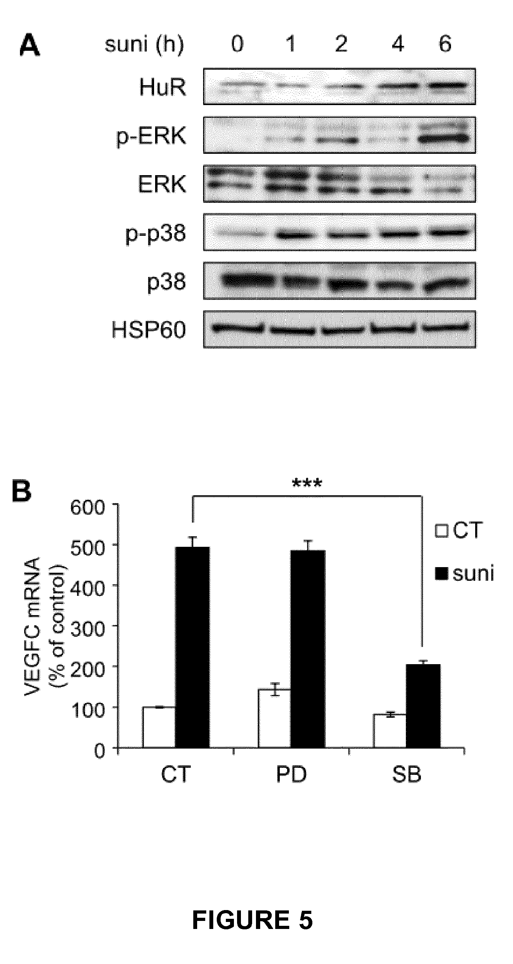

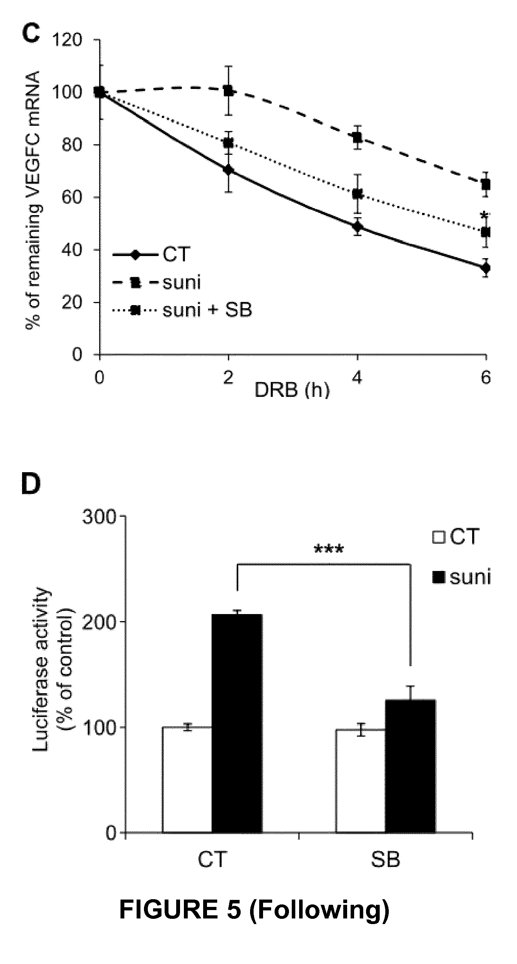

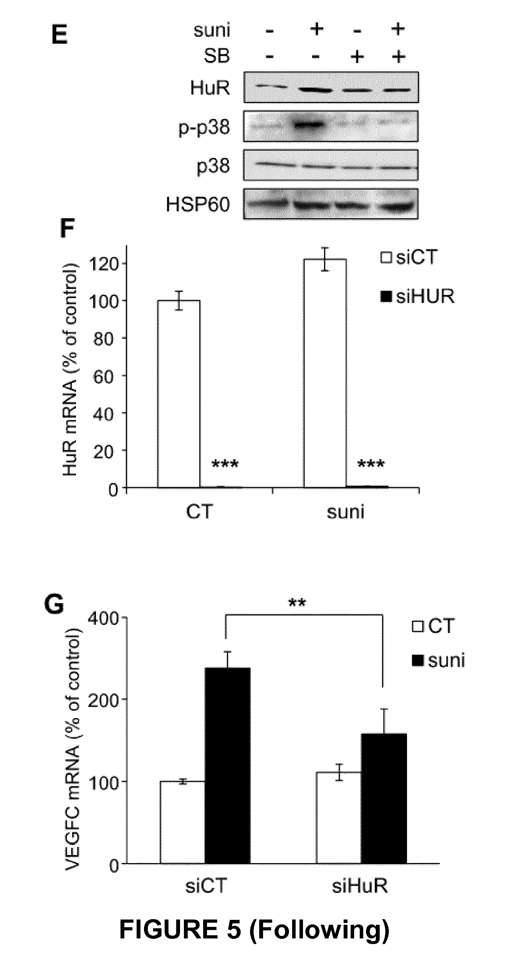

**Figure 5****: p38 and HuR were required for the induction of VEGFC expression by sunitinib.**
   **A**, 786-Ocells were treated with 5 µM sunitinib (suni) for 1 to 6 hr. HuR, p-ERK, ERK, p-p38 and p38 expression was analyzed by immunoblotting. HSP60 served as a loading control. **B,** 786-Ocells were treated with 5 µM sunitinib in the presence of 10 µM PD184352 (PD, ERK inhibitor) or 10 µM SB203580 (SB, p38 inhibitor) for 48 hr. The VEGFC mRNA level was determined by qPCR. **C**, 786-Ocells were treated with 5 µM sunitinib with or without 10 µM SB203580 for 48 hr. Cells were then treated with DRB for 2, 4 or 6 hr. The remaining VEGFC mRNA was evaluated by qPCR. **D,** 786-Ocells were transfected with a VEGFC luciferase 3'UTR reporter gene and treated or not with 5 µM sunitinib and/or with 10 µM SB203580 for 24 hr. The normalized luciferase counts served as a read out of the reporter gene mRNA half-life. **E,** 786-Ocells were treated with 5 µM sunitinib and/or 10 µM SB203580. HuR, p-p38 and p38 expression was analyzed by immunoblotting. HSP60 served as a loading control. **F** and **G,** 786-O cells were transfected with CT or HuR siRNA. 24 hr later, cells were treated with 5 µM sunitinib for 48 hr. HuR (**F**) and VEGFC (**G**) mRNA levels were determined by qPCR. Data are represented as mean of 3 independent experiments ± SEM. ** *p*<0.01, *** *p*<0.001. See also Figure 11.

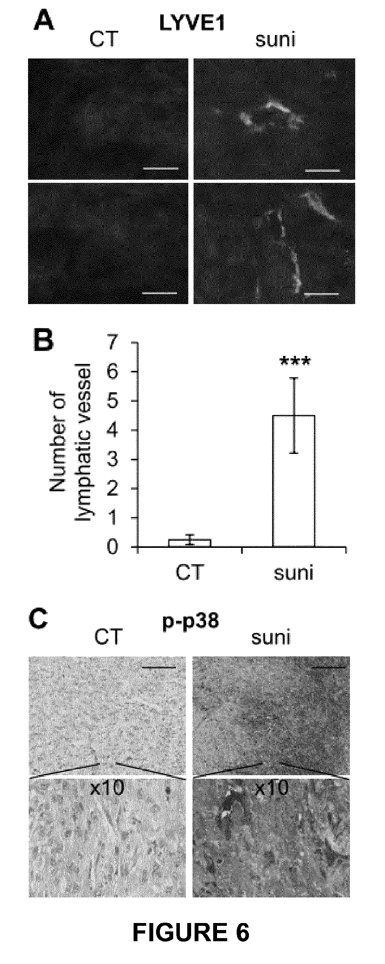

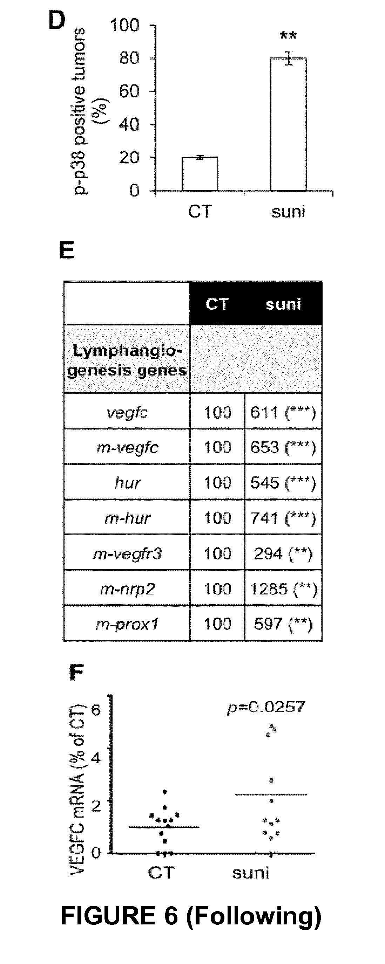

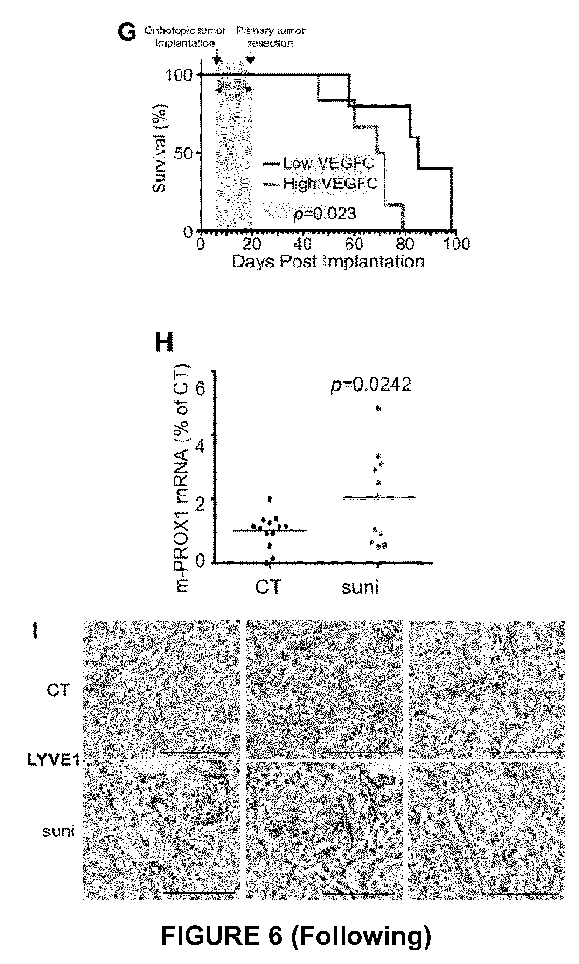

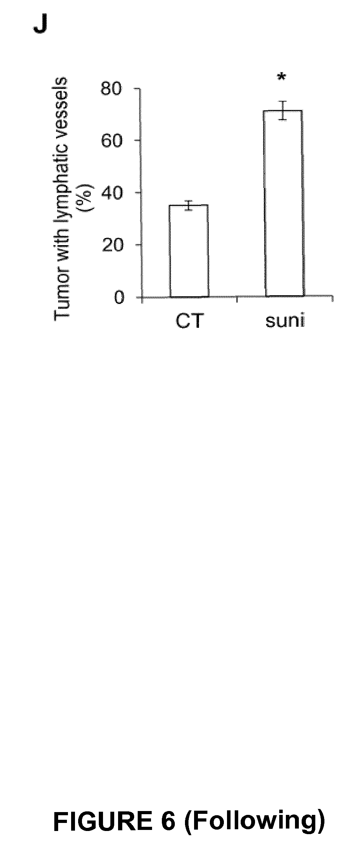

**Figure 6****: Sunitinib induced lymphangiogenesis in vivo.**
   Sunitinib induced lymphangiogenesis *in* vivo.**A**-**E**, experimental tumors obtained after subcutaneous injection of 5x10⁶ 786-Ocells were analyzed for LYVE1 expression. **A**, Lymphatic endothelial cells were detected by LYVE1 immuno-labeling (green). Scale bar: 20 µm. **B,** Quantification of the LYVE1 positive vessels. **C,** p-p38 was detected by immuno-histochemistry. Scale bar: 20 µm. **D,** Quantification of p-p38 labeled cells. **E,** The level of human (tumor cells) and mouse (stromal cells) mRNA were determined by qPCR. **F-J**, SN12-PM6^{LUC+} (2 × 10⁶ cells), were implanted into the kidney (subcapsular space) of 6- to 8-week-old female CB-17 SCID. **F,** VEGFC mRNA levels in tumors from control mice and mice treated in a neo-adjuvant setting, **G,** The Kaplan-Meier analysis of mice treated or not with sunitinib and the prognostic role of VEGFC mRNA levels. **H**, PROX1 mRNA levels in tumors from control mice and mice treated in a neo-adjuvant setting. **I,** Lymphatic endothelial cells were detected by LYVE1 immuno-histochemistry. Scale bar: 20 µm. **J,** Quantification of the LYVE1 positive vessels. ^{*} *p<0.05,* ** *p*<0.01, *** *p*<0.001.

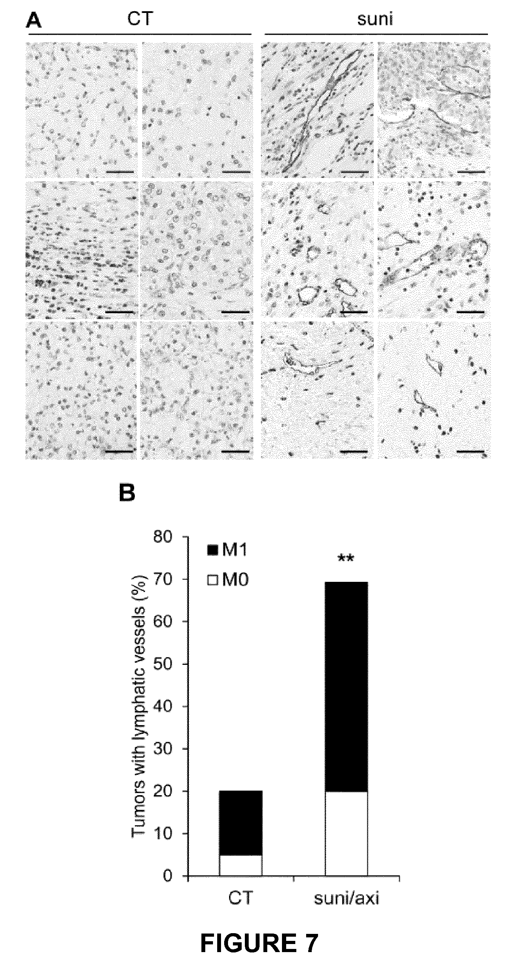

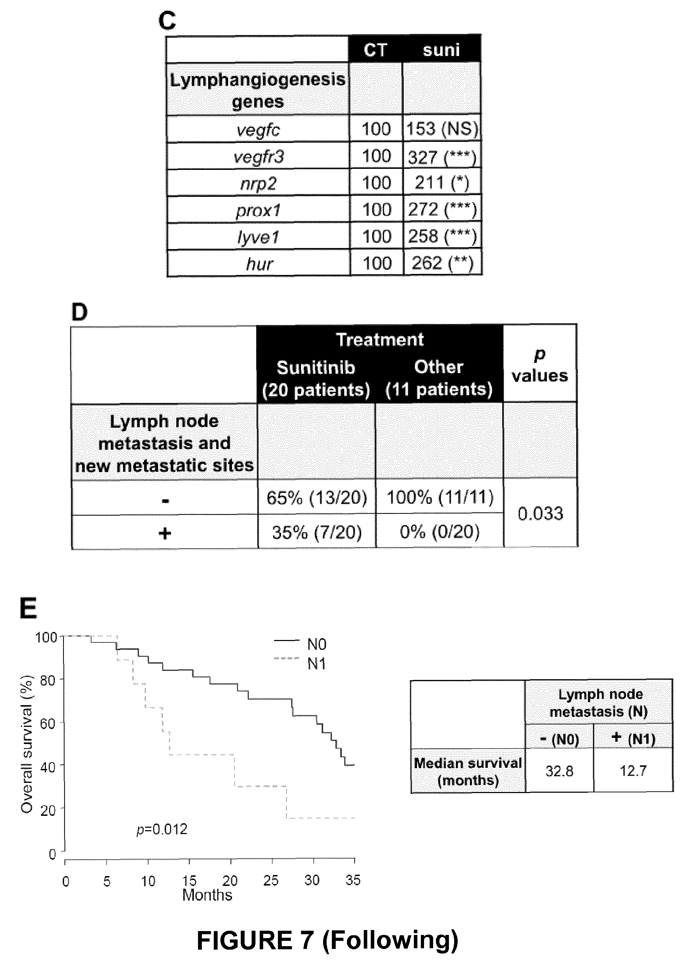

**Figure 7****: Sunitinib induced lymphangiogenesis and metastasis in lymph nodes of RCC patients.**
   Tumors from untreated RCC patients and tumors from patients treated with sunitinib or axitinib in a neo-adjuvant setting were compared (see Table 2). **A**, lymphatic endothelial cells were detected by podoplanin (PDN) immune-labeling (brown). Scale bar, 25 µM. **B**, Quantification of the PDN positive vessels. **C,** the levels of indicated mRNA were determined by qPCR. **D,** RCC patients that progressed on treatment were analyzed for the presence of lymph node metastasis and new metastatic sites (two or more new sites). Patients were stratified for sunitinib or other anti-angiogenic treatments (see Table 3). **E,** the Kaplan-Meier analysis of patients that had relapsed on sunitinib with non-invaded or *de novo* invaded lymph nodes. See also Figure 14.

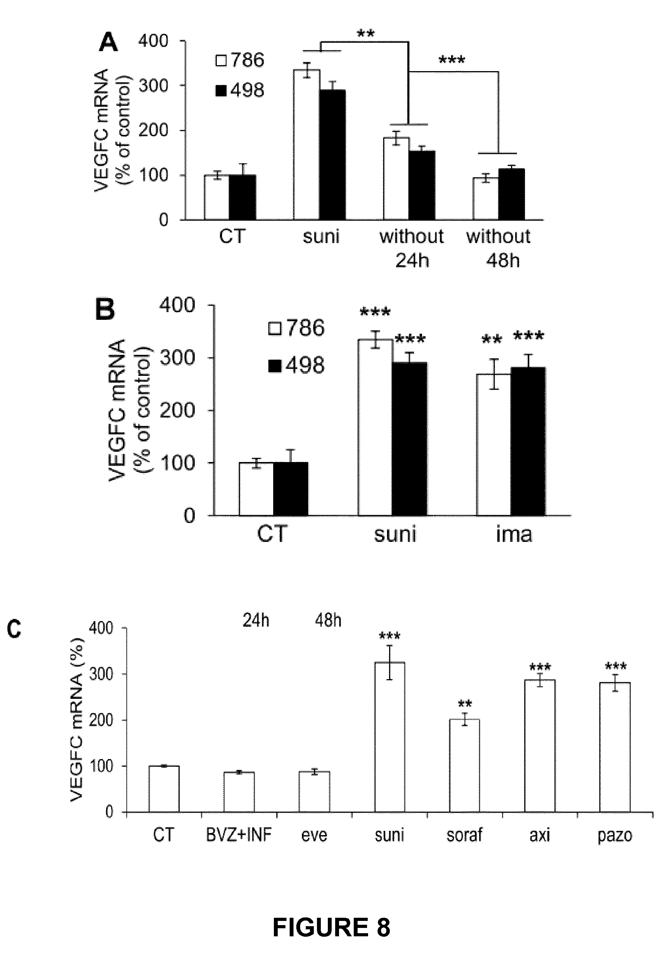

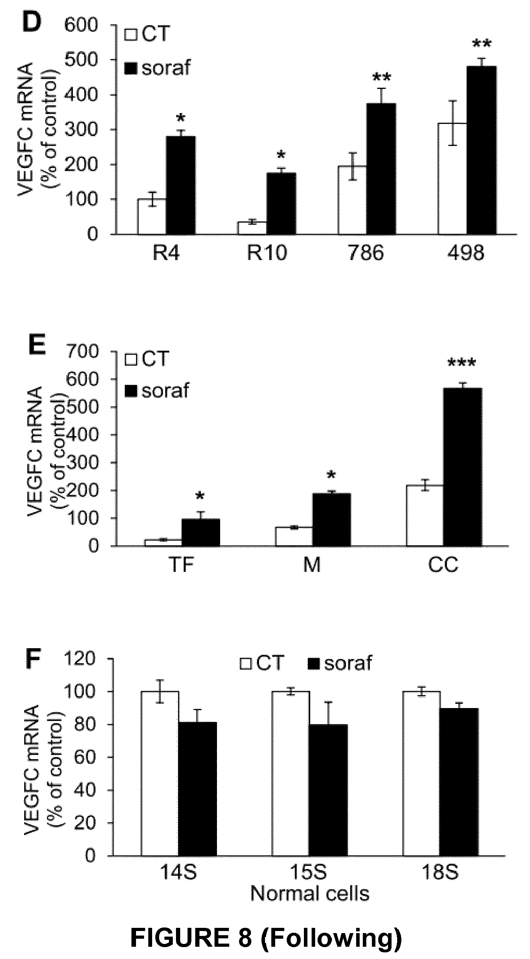

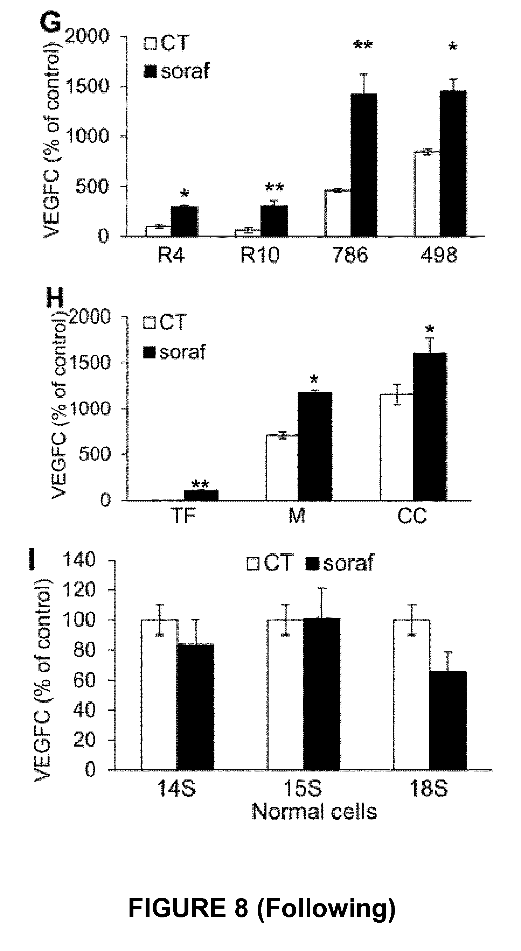

**Figure 8****: Tyrosine kinase Inhibitor stimulated VEGFC expression.**
   **A**, 786-O(786) and A498 (498) cells were treated with 5 µM sunitinib for 48 hr. The levels of VEGFC mRNA were evaluated by qPCR 24 or 48 hr after sunitinib removal. **B,** 786-O(786) and A498 (498) cells were treated with 5µM sunitinib or 10µM imatinib for 48 hr and the level of VEGFC mRNA was evaluated by qPCR. **C,** 786-0 cells were treated with 2 µg/ml bevacizumab + 1800 U/ml interferonα (BVZ+INF), 10 µM everolimus (eve), 5 µM sunitinib (suni), 5 µM sorafenib (soraf), 10 µM axtinib (axi), or 5 µM pazopanib (pazo) for 48 hr and the level of VEGFC mRNA was evaluated by qPCR. **D-I,** RCC cell lines [(RCC4 (R4), RCC10 (R10), 786-O(786) and A498 (498)] and primary RCC cells (TF, M and CC) and normal renal cells (14S, 15S and 18S) were evaluated for VEGFC mRNA levels by qPCR (**D-F**) and for VEGFC protein in cell supernatants by ELISA (**G-I**). RCC cell lines (**D**, **G**), primary cells (**E, H**) and normal kidney cells (**F**, **I**) were treated with 5 µM sorafenib for 48 hr. Data are represented as mean of 3 independent experiments ± SEM. * p<0.05, * * p<0.01, * ^{*} * p<0.001.
**Figure 9****: Overexpression of VEGFC in sunitinib resistant cells**
   **A**, 786-O(786) and 786-Oresistant's for sunitinib (786R) cells were treated with sunitinib for 48h and the cells viability were measured by XTT assay. **B,** 786-O(786) naive cells and 786-Ocells resistant to sunitinib (786R) were incubated in the presence of sunitinib (5 uM) and colored with Giemsa blue after 10 days (representative results of an experiment repeated trice). **C,** 786 and 786R were evaluated for VEGFC mRNA levels by qPCR. **D,** 786 and 786R were evaluated for VEGFC protein in cell supernatants by ELISA. **E,** p-p38 expression was analyzed by immunoblotting. p38 served as a loading control. **F,** 786 and 786R were transfected with a renilla luciferase reporter gene under the control of the VEGFC promoter. The renilla luciferase activity normalized to firefly luciferase (control vector) was the readout of the VEGFC promoter activity. **G,** 786 and 786R were transfected the VEGFC 3'UTR reporter gene. The level of renilla luciferase was normalized to the firefly luciferase as a readout of the modification of the reporter gene half-life. Data are represented as mean of three independent experiments ± SEM. * *p*<0.5, * * *p*<0.01, *** *p*<0.001.
**Figure 10****: Sorafenib stimulated VEGFC promoter activity and increased the VEGFC mRNA half-life.**
   **A** and **B,** RCC cell lines (**A**) and primary cells (**B**) were transfected with a renilla luciferase reporter gene under the control of the VEGFC promoter and treated with 5 µM sorafenib for 24 hr. The renilla luciferase activity normalized to firefly luciferase (control vector) was the readout of the VEGFC promoter activity. **C,** 786-Ocells were treated with 5 µM sorafenib for 48 hr. Cells were then incubated with DRB for 2, 4 or 6 hr. The remaining VEGFC mRNA was evaluated by qPCR. The VEGFC mRNA half-life was calculated. **D** and **E,** RCC cell lines (**D**) and primary cells (**E**) were transfected the VEGFC 3'UTR reporter gene and treated with 5 µM sorafenib for 24 hr. The level of renilla luciferase was normalized to the firefly luciferase as a readout of the modification of the reporter gene half-life. Data are represented as mean of 3 independent experiments ± SEM. **p*<0.5, * * *p*<0.01, *** *p*<0.001.
**Figure 11****: HuR and TTP bind the 3'UTR of VEGFC and VEGFA mRNA.**
   **A** and **B,** HuR/TTP interaction with VEGFA mRNA was analyzed by RIP-Chip. HEK293 cells were transfected with Myc-HuR or HA-CT (used as a negative control) (**A**) or with HA-TTP and Myc-CT (used as a negative control) (**B**). The levels of immunoprecipitated VEGFA or TNFα mRNA (used as positive controls) or GAPDH (used as a negative control) were determined by qPCR. Data are represented as mean of 3 independent experiments ± SEM. ** *p*<0.01.

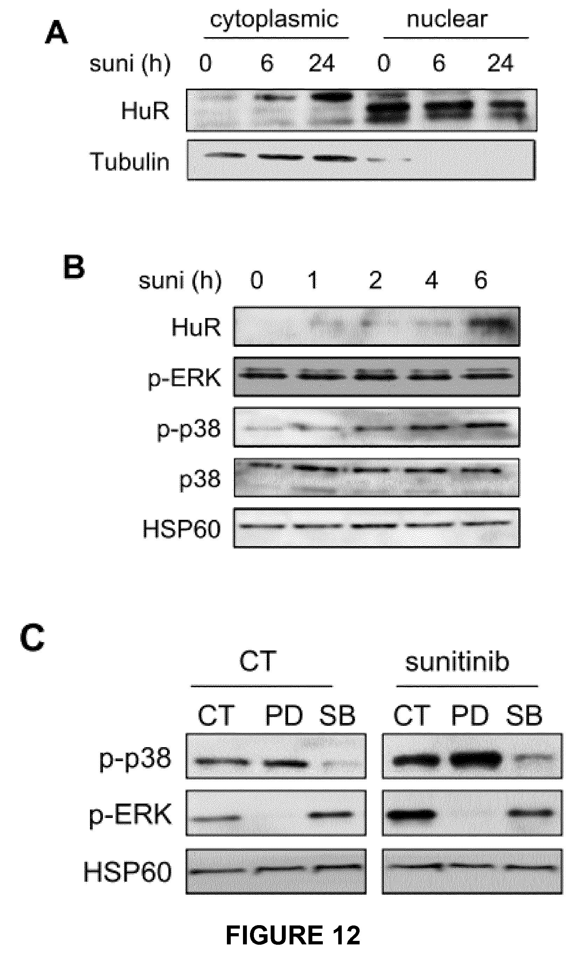

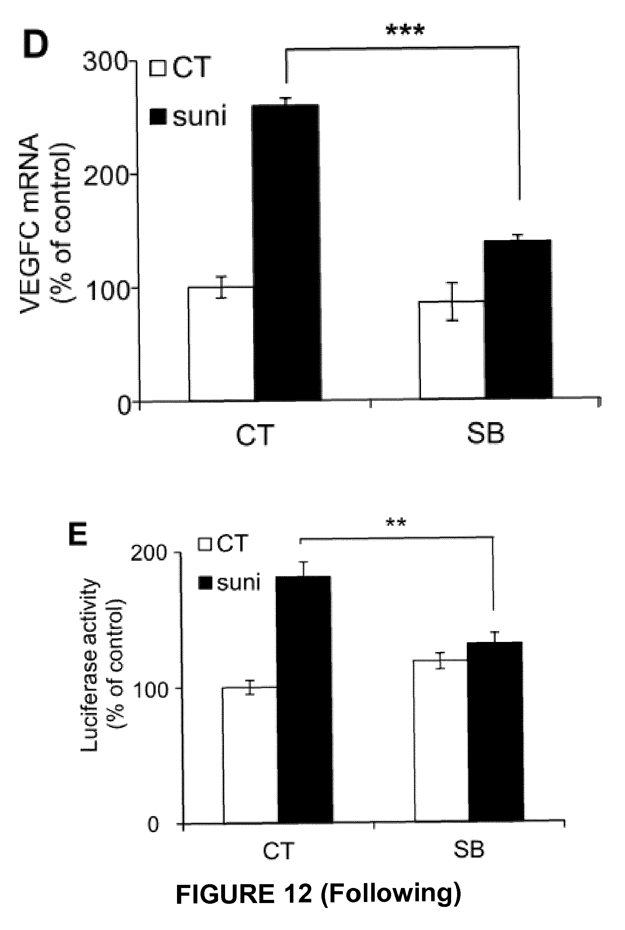

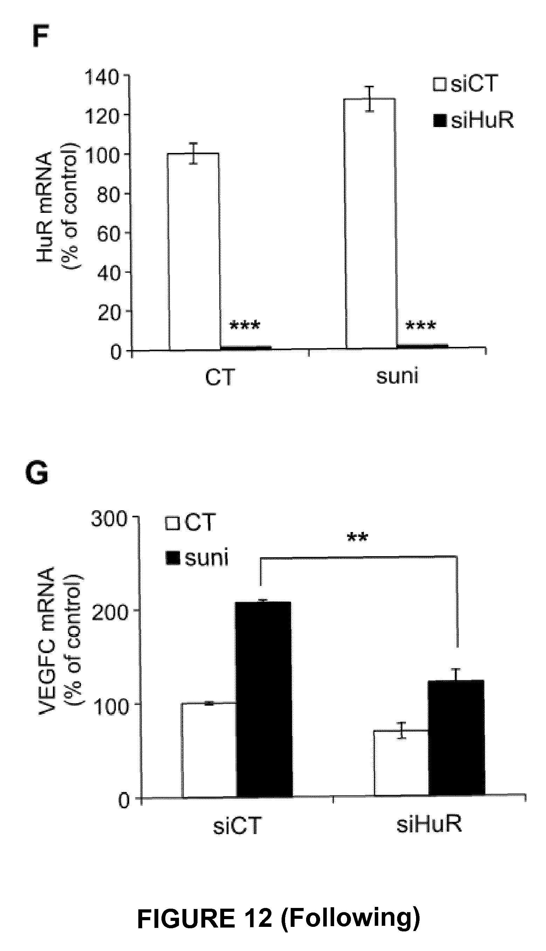

**Figure 12****: p38 and HuR were required for increased expression of VEGFC by sunitinib in RCC primary cells.**
   **A,** 786-Ocells were treated with 5 µM sunitinib (suni) for 6 hr or 24 hr. Cell extracts were separated into cytosol and nuclear-enriched fractions and HuR localization was determined by immunoblotting of the different fractions. Tubulin served as both a loading control and validation of the cytoplasmic fraction. **B,** primary cells (CC) were treated with 5 µM sunitinib for 1 to 6 hr. HuR, p-ERK, p-p38 and p38 expression was analyzed by immunoblotting. HSP60 served as a loading control. **C,** 786-Ocells were treated with 5 µM sunitinib in the presence of 10 µM PD184352 (PD) or 10 µM SB203580 (SB) for 4 hr. p-ERK, p-p38 expression was analyzed by immunoblotting. HSP60 served as a loading control. **D,** primary cells (CC) were treated with 5 µM sunitinib in the presence of 10 µM SB203580 for 48 hr. The VEGFC mRNA level was determined by qPCR. **E,** primary cells (CC) were transfected with VEGFC luciferase 3'UTR reporter gene and treated or not with 5 µM sunitinib and/or with 10 µM SB203580 for 24 hr. The normalized luciferase counts served as readout of the reporter gene mRNA half-life. **F** and **G,** primary cells (CC) were transfected with CT or HuR siRNA. 24 hr later, cells were treated with 5 µM sunitinib for 48 hr. HuR (**F**) and VEGFC (**G**) mRNA levels were determined by qPCR. Data are represented as mean of 3 independent experiments ± SEM. * * *p*<0.01, *** *p*<0.001.

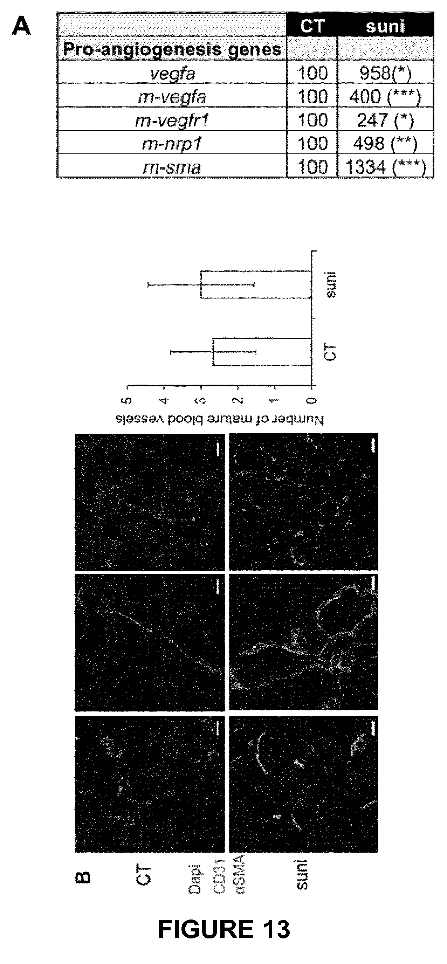

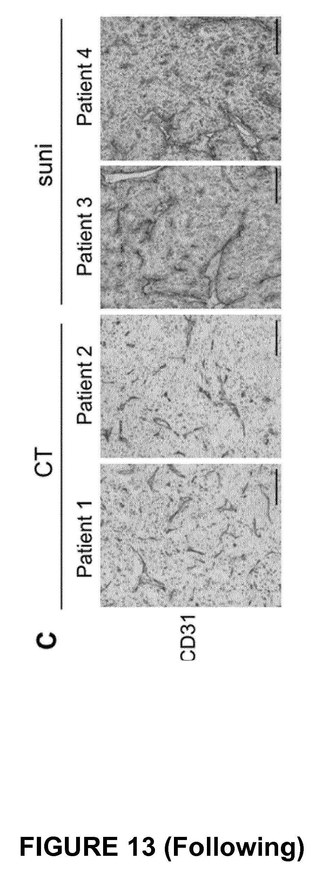

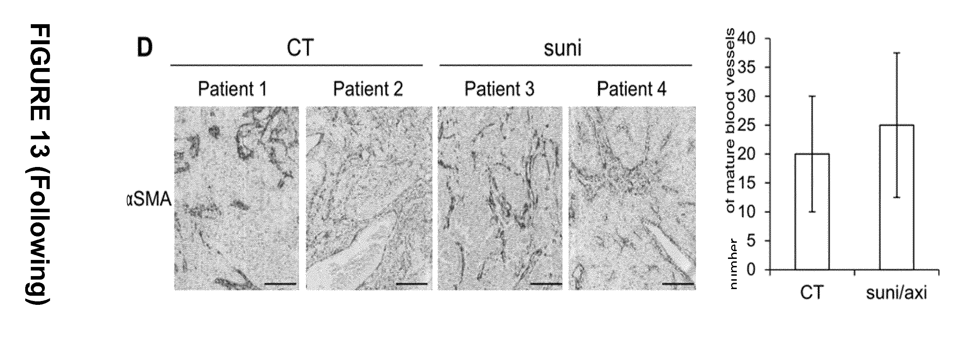

**Figure 13****: Sunitinib does not modified blood vessels.**
   Experimental tumors obtained by subcutaneous injection of 5.10⁶ 786-O cells in mice. **A,** The level of human (produced by tumor cells) and murine (m, produced by mouse stromal cells) mRNA were determined by qPCR (* p<0.05, ** *p*<0.01, *** *p*<0.001. **B,** The tumor vasculature was detected by CD31 (endothelial cells, green) and α-SMA immuno-staining (pericytes, red). Tumor sections were counterstained with Dapi (nuclei, blue). Scale bar: 20 µM. Quantification of the number of vessels showing with co-localization of CD31 and a-SMA is shown on the right. **C** and **D,** tumors from RCC patients treated or not with sunitinib (suni) in a neoadjuvant setting. The tumor vasculature was detected by immuno-staining for CD31 (**C**, endothelial cells) and α-SMA (**D**, pericytes). Scale bar: 100 µM. The quantification of the number of CD31 and a-SMA labeled vessels is shown.
**Figure 14****: Overexpression of VEGFC correlated with reduced overall survival of RCC patients.**
   **A**, kaplan-Meier survival curves of non-metastatic (M0) and metastatic (M1) RCC patients with high or low VEGFC mRNA expression. These results are in whole or in part based upon data generated by the TCGA Research Network. **B,** proportion of groups of patients with VEGFC low or high and its correlation with metastatic stage (M1). Statistical significance (Khi2 test) is indicated. **C,** Multivariate analysis for VEGFC, stage 3/4, metastatic stage (M1) and lymph node metastatic stage (N1). Statistical significance (p values) and the risk ratio are indicated.
**Figure 15****: Recapitulatve schema of VEGFC-mediated induction by anti-angiogenic drugs and or chemotherapeutic drugs inducing p38 activation.**

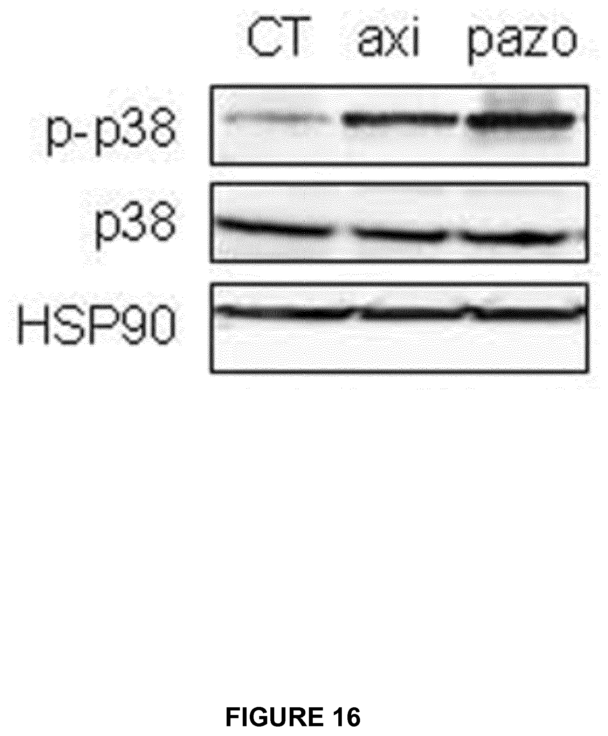

**Figure 16****:** 786-Ocells were treated with 5 µM sunitinib, 10µM axitinib or 10µM pazopanib for 4 hr. p-p38 and p38 expression was analyzed by immunoblotting. HSP90 served as a loading control.

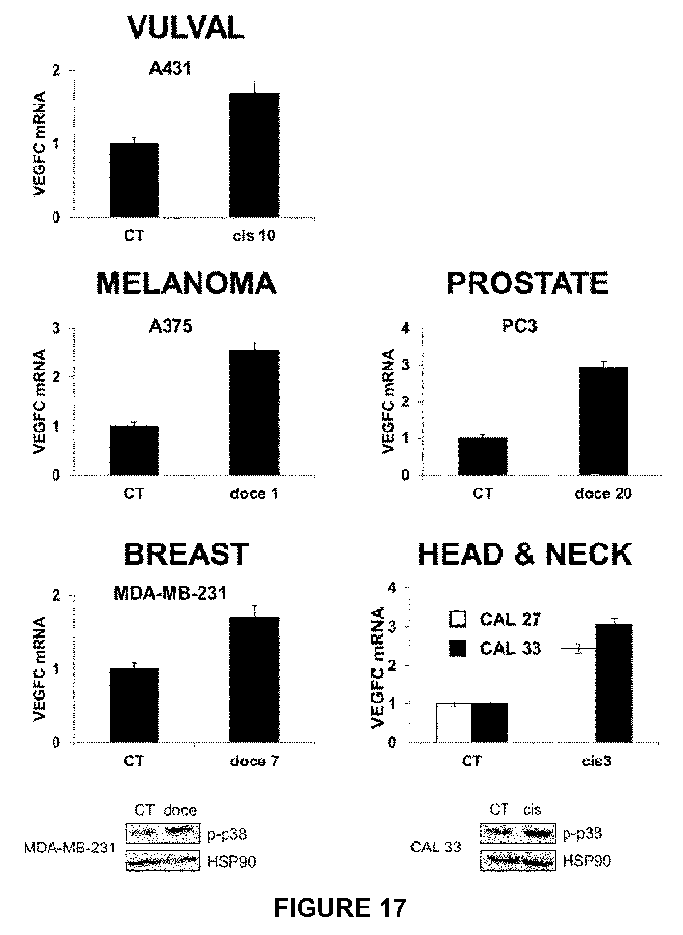

**Figure 17****: VEGFC induction by standard/conventional chemotherapy in different cancers.**
   Model cell lines for metastatic cancers including breast cancer (MDA MB 231), prostate cancer (PC3), melanoma (A375), vulval cancer (A431) and head and neck cancer (CAL 27 and CAL 33) were treated with docetaxel (doce) or cisplatine (cis) for the indicated concentrations. These concentrations correspond to the IC50 doses as described in the literature. For docetaxel, concentrations are expressed in nanomolar and for cisplatine in micromolar. For each breast and head and neck cancer cell lines p-p38 was analyzed by immunoblotting. HSP90 served as a loading control.

### DETAILED DESCRIPTION OF THE INVENTION

Sunitinib, axitinib, pazopanib and imatinib are antiangiogenic therapies given as a first line treatment for renal cell carcinoma (RCC). While treatment improves progression free-survival, most patients relapse. Inventors hypothesized that patient relapse can stem from the development of a lymphatic network driven by the production of the main growth factor for lymphatic endothelial cells, VEGFC.

Working on RCC, inventors have herein discovered an ordered sequence of molecular events in the pathway leading to the occurrence (development) of new metastasis(es) or the development of existing metastasis(es) in a subject suffering of a cancer.

They discovered that in particular sunitinib, axitinib, pazopanib, imatinib as well as taxans and platinum salts (also herein identified as "platins") activate p38 MAP Kinase which resulted in the up-regulation/activity of HuR and inactivation of tristetraprolin, two AU-rich element binding proteins, and can stimulate *vegfc* gene transcription and increase VEGFC mRNA half-life. Furthermore, they showed that sunitinib, axitinib, pazopanib, imatinib, taxans and platins stimulate a VEGFC-dependent development of lymphatic vessels in experimental tumors, and established a link with their findings of increased lymph node invasion and new metastatic sites in 30% of patients treated with a treatment inducing p38 activation, and increased lymphatic vessels found in 70% of neoadjuvant treated patients. They conclude that a therapy dedicated to destroying tumor blood vessels such as sunitinib, axitinib, pazopanib and imatinib in particular induced the development of lymphatic vessels which contribute to treatment failure.

Inventors herein reveal for the first time that detection of p38 activation in a subject can be used to determine if the subject will develop or not new or existing metastasis when exposed to a particular treatment.

Inventors also herein advantageously describe for the first time an anti-VEGF antibody, or a functional fragment thereof, for use for preventing the occurrence of new metastases or the development of existing metastases in a subject exposed to or who will be exposed to a cancer treatment inducing p38 activation. Inventors herein reveal that a treatment selected from axitinib, pazopanib, imatinib, taxans and platins in particular induces p38 activation, and as a consequence, is responsible for the development of new or of existing metastasis in the subject. At the same time, they reveal that antiangiogenic factors, distinct in particular from axitinib, pazopanib and imatinib, such as regorafenib, do not induce p38 activation and do not induce the development of new or existing metastases.

The term "angiogenic factor or agent" refers to a growth factor or its receptor which is involved in stimulating the development of blood vessels, e.g., promoting angiogenesis, endothelial cell growth, stability of blood vessels, and/or vasculogenesis, etc.

In the context of the invention, the terms "antiangiogenic factor inducing p38 activation" preferably designate an angiogenic factor preferably selected from axitinib, pazopanib and imatinib. A particularly preferred antiangiogenic factor inducing p38 activation is axitinib.

Taxanes or taxoids are based on taxol (paclitaxel) which is derived from the bark of the Pacific Yew tree. They work by enhancing stability of microtubules thereby preventing separation of chromosomes during anaphase. Examples of taxanes/taxoids include paclitaxel (TAXOL® paclitaxel), abraxane (ABRAXANE™ Cremophor-free, albumin-engineered nanoparticle formulation of paclitaxel) and docetaxel (TAXOTERE® doxetaxel). In the context of the invention, the terms "taxan inducing p38 activation" designates a taxan selected from docetaxel, paclitaxel and cabazitaxel. A preferred taxan inducing p38 activation is docetaxel.

In the context of the invention, the terms "platinum salt, or "platin", inducing p38 activation" preferably designates a platin selected from cisplatin, carboplatin and oxaliplatin. A particularly preferred platin inducing p38 activation is cisplatin.

In the context of the present invention, the patient or subject is a mammal. The mammal may be a primate, particularly a human being (also herein identified as "human"), or may be a domestic mammal such as horses, cattle and sheep, a companion animal such as dogs and cats, or a zoo mammal such as felids, canids, bovids and ungulates.

In a preferred embodiment, the mammal is a human being, whatever its age or sex. Unless otherwise specified in the present disclosure, the subject has a tumor or cancer, typically a malignant tumor, in particular a metastatic (malignant) tumor.

In a particular embodiment of the present invention, the subject is typically a subject undergoing a treatment of cancer, in particular a conventional treatment of cancer (for example chemotherapy and/or radiotherapy). The term "conventionally" means that the therapy is applied or, if not routinely applied, is appropriate and at least recommended by health authorities. The "conventional" treatment is selected by the cancerologist depending on the specific cancer to be prevented or treated.

In the context of the present invention, a "conventional treatment of cancer" may be selected from a chemotherapy, a radiotherapy, an hormonotherapy, an immunotherapy, a specific kinase inhibitor-based therapy, an antiangiogenic agent based-therapy, an antibody-based therapy, in particular a monoclonal antibody-based therapy, and surgery.

The subject may have been exposed to only part of a complete conventional treatment protocol, for example to at least one cycle of the all treatment protocol, for example two cycles of the all treatment protocol.

When exposed to cancer treatment inducing p38 activation, the subject will typically develop lymphatic vessels and ultimately (cancer) metastases. Typically, the appearance of the first metastasis does not immediately follow the administration of the cancer treatment inducing p38 activation. The first metastasis typically appears between 6 and 12 months, for example 7, 8, 9, 10 or 11 months, following the administration of the first cancer treatment inducing p38 activation.

In another particular embodiment of the present invention, the subject has never been exposed to a treatment of cancer.

In a particular aspect, the subject has a tumor requiring, according to physicians (typically oncologists or cancerologist), a neoadjuvant cancer treatment before practicing any tumor or metastase surgical resection on said subject. Thus, in a particular embodiment, the subject is a subject who is exposed to, or a subject who will be exposed to, a neoadjuvant cancer treatment, typically a neoadjuvant cancer treatment inducing p38 activation as herein described, preferably a neoadjuvant anti-angiogenic treatment inducing p38 activation selected from axitinib, pazopanib and imatinib. In the context of the invention, a preferred neoadjuvant cancer treatment inducing p38 activation is axitinib.

In the context of the present invention, the cancer or "malignant tumor" may be any kind of cancer or neoplasia, typically metastatic cancer or neoplasia. The cancer or tumor can be selected from a carcinoma, a sarcoma, a melanoma and a pediatric tumour. The cancer is preferably selected from a renal or kidney cancer, preferably a renal cancer carcinoma (RCC), in particular a clear cell renal cancer carcinoma (ccRCC); a breast cancer; an ovarian cancer; a lung cancer, in particular a non-small cell lung cancer (NSCLC); a melanoma; a head and neck cancer; a colon cancer; and a mesothelioma. The cancer is for example a renal cancer carcinoma (RCC), in particular a clear cell renal cancer carcinoma (ccRCC).

Herein described is an anti-VEGF antibody, or a functional fragment thereof, for use for preventing the occurrence of new metastases or the development of existing metastases in a subject exposed to or who will be exposed to a cancer treatment inducing p38 activation, wherein the cancer treatment is part of a treatment protocol comprising a step of assessing p38 activation in at least one metastasis or tumor cell, in particular metastatic cell, of the subject, optionally together with a step of assessing the presence of lymphatic vessels, typically of an excessive/atypical amount of lymphatic vessels, when compared typically to that of non-metastatic primary tumor.

It is to be understood that the expression "tumor cells" used to identify cells obtained from a tumor of a subject, is used, in the present description, to identify circulating tumor cells, cells obtained from a tumor bed, or cells obtained from a metastasis.

In a particular embodiment, the step of assessing p38 activation in a subject occurs before the administration of any cancer treatment known to induce p38 activation to the subject, typically in a subject who has been already exposed to a conventional treatment of cancer, for example a treatment which is not known to induce p38 activation.

The step of assessing p38 activation preferably occurs after the administration to the subject, typically after the first administration to the subject, of a cancer treatment inducing p38 activation.

When performed after the first administration to the subject of a cancer treatment inducing p38 activation, the step of assessing p38 activation typically occurs about 4 to 12 months, typically 6 to 12 months, for example 5, 6, 7, 8, 9, 10 or 11 months, following said first administration of the cancer treatment inducing p38 activation to the subject and/or when a 5 to 20% increase, for example 6, 7, 8, 9, 10 or 15% increase, of the volume of the primary tumor or of at least one metastasis, preferably of at least one metastasis, typically several metastasis, for example 2, 3, 4, 5 or 6 metastases, has been measured since the first administration of the cancer treatment inducing p38 activation. The assessed/measured metastasis(es) can be any primary, secondary or subsequent metastasis(es) originating from a primary (cancerous) tumor. The terms "primary metastasis" encompasses distant metastasis as well as local invasion of a primary tumor. A typical "distant" metastasis is a hepatic, lung, brain, skin or spinal metastasis.

P38 is considered to be activated when it is in a phosphorylated form. The step of assessing p38 activation thus typically involves the detection of a phosphorylated version of p38 (also herein identified as phospho-p38 or p-p38). Such detection can be carried out easily by immunostaining using an anti-phospho-p38 antibody, typically a commercially available antibody (such as ref.: 4631 from Cell Signaling Technology, Beverly, MA, USA). As well known by the skilled person, a detectable secondary antibody binding the anti-phospho-p38 antibody is typically used to concretely detect/measure p38 activation (cf. experimental part). An additional anti-total p38 antibody (linking both p38 and phospo-p38, typically the C-terminal part of p38) can further be used in order to measure p38 activation.

The assessment of p38 activation can also be carried out easily by immunoblot as well known by the skilled person (Mueller et al., 2011). Typically the presence/amount of both p-p38 and p38 in total lysate of tumor homogenates can be assessed using immunoblot.

The volume/size of a metastasis or tumor can be determined/measured using conventional means/methods of the art, well known by the skilled person, such as imaging means/methods, typically Computed tomography scanning (CT) (Grépin et al. Plos 2014).

It is inventors' belief that the step of assessing p38 activation could complete the RECIST (Response Evaluation Criteria in Solid Tumor) criteria set for assessment of tumor as it allows the detection of an intermediate tumor profile between tumor that respond to treatment ("responding tumors") and tumor that worsen during treatment ("non-responding tumor" or "progressing tumor"), in addition to tumor that stay the same ("stabilized tumor"), and most important, a solution to avoid patients to move from the first category ("responder patient" or "sensitive patient") to the second ("non-responder patient" or "resistant patient").

Inventors indeed now advantageously describe an anti-VEGFC antibody, or a functional fragment thereof (i.e. a fragment retaining the ability to bind antigen), for use for preventing the occurrence of new metastases or the development of existing metastases in a subject exposed to or who will be exposed to a cancer treatment inducing p38 activation, preferably selected from axitinib, pazopanib, imatinib, a taxan and a platin. The term "antibody fragment" refers to a molecule comprising only a portion of an intact antibody, generally including an antigen binding site of the intact antibody and thus retaining the ability to bind antigen. Examples of antibody fragments encompassed by the present definition include: (i) the Fab fragment, having VL, CL, VH and CH1 domains; (ii) the Fab' fragment, which is a Fab fragment having one or more cysteine residues at the C- terminus of the CH 1 domain; (iii) the Fd fragment having VH and CH 1 domains; (iv) the Fd' fragment having VH and CH 1 domains and one or more cysteine residues at the C-terminus of the CH 1 domain; (v) the Fv fragment having the VL and VH domains of a single arm of an antibody; (vi) the dAb fragment which consists of a VH domain; (vii) isolated complementarity determining regions (CDRs); (viii) F(ab')2 fragments, a bivalent fragment including two Fab' fragments linked by a disulphide bridge at the hinge region; (ix) single chain antibody molecules (e.g. single chain Fv; scFv); (x) "diabodies" with two antigen binding sites, comprising a heavy chain variable domain (VH) connected to a light chain variable domain (VL) in the same polypeptide chain; (xi) "linear antibodies" comprising a pair of tandem Fd segments (VH-CH 1 -VH-CH 1) which, together with complementary light chain polypeptides, form a pair of antigen binding regions.

Anti-VEGFC antibodies are available and described in the art.

An example of anti-VEGFC antibody which can be used in the context of the invention is identified as "VGX-100" in WO2011/127519 [see figure 3 of WO2011/127519 wherein the heavy chain variable (VH) region is underlined, and figure 4 of WO2011/127519 wherein the light chain variable (VL) region is underlined].

"VGX-100" is defined as having SEQ ID NO: 9 as heavy chain and SEQ ID NO: 10 as light chain. SEQ ID NO: 11 designates the heavy chain variable (VH) region of "VGX-100" and SEQ ID NO: 12 designates the light chain variable (VL) region of "VGX-100".

Other examples of anti-VEGFC antibodies are described in WO2011/071577, such as in particular those respectively identified as "VC4.5" and "VC1.12" which can, among others, be used in the context of the invention.

"VC4.5" is defined as having SEQ ID NO: 13 as heavy chain and SEQ ID NO: 14 as light chain. "VC4.5" can otherwise be defined as having the six following CDRs: SEQ ID NO: 15 (CDR1), SEQ ID NO: 16 (CDR2) and SEQ ID NO: 17 (CDR3) corresponding to the CDRs of the light chain of "VC4.5", and SEQ ID NO: 18 (CDR1), SEQ ID NO: 19 (CDR2) and SEQ ID NO: 20 (CDR3) corresponding to the CDRs of the heavy chain of "VC4.5".

"VC1.12" is defined as having SEQ ID NO: 21 as heavy chain and SEQ ID NO: 22 as light chain.

"VC1.12" can otherwise be defined as having the six following CDRs: SEQ ID NO: 23 (CDR1), SEQ ID NO: 24 (CDR2) and SEQ ID NO: 25 (CDR3) corresponding to the CDRs of the light chain of "VC1.12", and SEQ ID NO: 26 (CDR1), SEQ ID NO: 27 (CDR2) and SEQ ID NO: 28 (CDR3) corresponding to the CDRs of the heavy chain of "VC1.12".

In a typical embodiment of the invention, the cancer treatment inducing p38 activation and the anti-VEGFC antibody are administered sequentially, the anti-VEGFC antibody being administered after the cancer treatment inducing p38 activation, typically after the first cancer treatment inducing p38 activation, preferably when p38 activation has been detected in at least one tumor cell of the subject, typically in at least one metastasis or metastatic cell of the subject.

In a particular embodiment, the anti-VEGFC antibody is administered to the subject in need thereof together with the cancer treatment inducing p38 activation (simultaneous administration), typically when said treatment is not the first cancer treatment inducing p38 activation administered to the subject but is, for example, a second or subsequent treatment inducing p38 activation administered to the subject.

The anti-VEGFC antibody is typically administered to the subject in need thereof after at least one step of assessing p38 activation has been performed and p38 activation has been detected in said subject.

The anti-VEGFC antibody administration can be performed immediately following the p38 activation detection in combination with the treatment inducing p38 activation, which is typically the treatment of reference (also herein identified as conventional treatment) administrated once the disease has been diagnosed. It can also be administered with a second distinct recommended cancer treatment ("second line treatment") which is part of cancer treatment protocol, said second distinct cancer treatment being typically selected from axitinib, pazopanib, imatinib, preferably together with axitinib or pazopanib.

A further object herein described is a cancer treatment inducing p38 activation, typically selected from axitinib, pazopanib, imatinib, a taxan and a platin, for use in combination with an anti-VEGFC antibody, or a functional fragment thereof, for preventing the occurrence of new metastases or the development of existing metastases in a subject, preferably for use sequentially, the anti-VEGFC antibody being typically administered after the cancer treatment inducing p38 activation.

In a particular aspect, the anti-VEGFC antibody, or a functional fragment thereof, is administered to the subject in need thereof together with (simultaneous administration) the cancer treatment inducing p38 activation (as a combined product), typically when said treatment is not the first cancer treatment inducing p38 activation administered to the subject but is, for example, a second or subsequent treatment inducing p38 activation administered to the subject.

In a particular aspect, the cancer treatment inducing p38 activation is for use in combination with an anti-VEGFC antibody, or a functional fragment thereof, for treating a cancer in a subject in need thereof and preventing the development of metastasis(es) in said subject.

Also herein described are a method for preventing the occurrence of new metastases or the development of existing metastases in a subject as herein defined or a method for treating cancer in a subject in need thereof and preventing the development of metastasis(es) in said subject, wherein the method comprises a step of administering simultaneously or sequentially a cancer treatment inducing p38 activation and an anti-VEGFC antibody, or a functional fragment thereof, the anti-VEGFC antibody or functional fragment thereof being typically administered after, or together with the cancer treatment inducing p38 activation.

The terms "treat", "treating" or "treatment" refer to both therapeutic treatment and prophylactic or preventative measures, wherein the aim is to prevent or ameliorate cancer or slow down (lessen) cancer progression. Those in need of treatment include those already with the disorder as well as those in which the disorder is to be prevented.

The terms "preventing", "prevention", "preventative" or "prophylactic" refers to keeping from occurring, or to hinder, defend from, or protect from the occurrence of a condition, disease, disorder, or phenotype, including an abnormality or symptom. A subject in need of prevention may be prone to develop the condition.

The anti-VEGFC antibody (or fragment thereof) administration lasts at least throughout the cancer treatment inducing p38 activation administration period and can be extended later for a period which may vary depending on the selected cancer treatment inducing p38 activation and/or on the treated subject, and which can be of several days, weeks or months.

In a particular embodiment, the anti-VEGFC antibody (or fragment thereof) administration lasts at least throughout the cancer treatment inducing p38 activation administration period and can be extended later for a period which ends in case of patient relapse.

In the context of the invention, the anti-VEGFC antibody, or functional fragment thereof, is administered to the subject in a therapeutically effective amount [amount effective to reduce the number of cancer cells, kill cancer cells, reduce the tumor volume/size, or inhibit tumor growth, when the anti-VEGFC antibody is used in combination with the cancer treatment inducing p38 activation] or prophylactically effective amount [amount effective to reduce or suppress lymphatic vessels formation or development and/or reduce or prevent the occurrence of new metastasis(es) or the development of existing metastasis(es)], typically at a concentration range from about 2 to 20 mg/kg of body weight, preferably from about 5 to 15 mg/kg of body weight, for example 6, 7, 8, 9, 10, 11, 12, 13 or 14 mg/kg of body weight, preferably 10 mg/kg of body weight.

For cancer therapy, efficacy *in vivo* can, for example, be measured by assessing the duration of survival, time to disease progression, the response rates, duration of response, and/or quality of life.

In a particular aspect of the invention, when the cancer treatment inducing p38 activation is an antiangiogenic factor, the anti-VEGFC antibody (or functional fragment thereof) is typically administered to a human being at a concentration range from about 2 to 20 mg/kg of body weight, preferably 5 to 15 mg/kg of body weight.

For example, when the cancer treatment inducing p38 activation is selected from axitinib, pazopanib and imatinib, and is in particular axitinib, the anti-VEGFC antibody (or functional fragment thereof) is typically administered to a human being at a concentration range from about 5 to 15 mg/kg of body weight, preferably 6, 7, 8, 9, 10, 11, 12, 13 or 14 mg/kg of body weight, even more preferably 10 mg/kg of body weight. In another particular aspect of the invention, when the cancer treatment inducing p38 activation is a taxan, the anti-VEGFC antibody (or functional fragment thereof) is typically administered to a human being at a concentration range from about 5 to 15 mg/kg of body weight.

In another particular aspect of the invention, when the cancer treatment inducing p38 activation is a platin, the anti-VEGFC antibody (or functional fragment thereof) is typically administered to a human being at a concentration range from about 5 to 15 mg/kg of body weight.

A further object herein described is a method for selecting, typically *in vitro* or *ex vivo,* an optimal therapeutic treatment of cancer in a subject having metastasis(es) exposed to a cancer treatment inducing p38 activation, preferably selected from axitinib, pazopanib, imatinib, a taxan and a platin, wherein the method comprises a step of assessing p38 activation *in vitro* or *ex vivo* in at least one metastasis or metastatic cell of the subject as described previously, and a step of deciding that the cancer treatment inducing p38 activation is to be combined to the administration to the subject of an anti-VEGFC antibody (or functional fragment thereof) as the (combined) optimal therapeutic treatment of cancer for the subject, preferably if an activation of p38 is detected in the at least one metastasis or metastatic cell of the subject.

Reference to administration "in combination" refers to simultaneous (concurrent) and consecutive administration in any order. The combined administration includes coadministration, using separate formulations or a single pharmaceutical formulation, and consecutive administration in any order.

Thanks to the present invention, the subject receiving the combined treatment will be completely treated (cured), i.e., the subject will survive to the cancer [beneficial impact on the "overall survival" (OS)], or will have a much longer disease free survival (DFS) or metastasis free survival chance than a patient who does not receive said combined treatment.

A further object herein described is a kit, typically a prophylactic and/or therapeutic kit, comprising (i) a cancer treatment inducing p38 activation, typically selected from axitinib, pazopanib, imatinib, a taxan and a platin, and (ii) an anti-VEGFC antibody, or functional fragment thereof, in particular a specific anti-VEGFC antibody (or functional fragment thereof) as herein described.

Inventors also herein describe the advantageous use of such a kit for preventing the occurrence of new metastasis(es) or the development of existing metastasis(es) in a subject, in particular for treating cancer and preventing the occurrence of new metastasis(es) or the development of existing metastasis(es) in a subject.

A particular object of the invention is thus a kit as herein described for use for treating cancer and preventing the occurrence of new metastasis(es) or the development of existing metastasis(es) in a subject.

Further herein described is a kit, comprising at least one anti-p38 antibody for detecting p38 activation in at least one metastasis or metastatic cell from a subject suffering of a cancer, and at least one secondary antibody detecting the at least one anti-p38 antibody. The at least one anti-p38 antibody for detecting p38 activation is typically an anti-phospho-p38 (anti-p-p38) antibody, for example an anti-p-p38 antibody as described in the herein below detailed experimental part. The at least one secondary antibody is typically a detectable secondary antibody binding the anti-phospho-p38 antibody.

The kit can further comprise in addition an anti-total p38 antibody, said antibody linking both p38 and phospo-p38, typically the C-terminal part of p38.

The herein above described kits can optionally comprise in addition an anti-VEGFC antibody (or functional fragment thereof), such as an anti-VEGFC antibody as herein described.

Inventors herein describe a kit as previously described (comprising at least one anti-p38 antibody for detecting p38 activation in at least one metastasis or metastatic cell from a subject suffering of a cancer, and at least one secondary antibody detecting the at least one anti-p38 antibody) for use in a method as herein described, typically for use for selecting, typically *in vitro* or *ex vivo,* an optimal therapeutic treatment of cancer in a subject having metastasis(es) exposed to a cancer treatment inducing p38 activation, preferably selected from axitinib, pazopanib, imatinib, a taxan and a platin.

Further aspects and advantages of the present invention will be described in the following examples, which should be regarded as illustrative and not limiting. All references cited in the present application are herein incorporated by reference.

### EXAMPLES

### EXAMPLE 1: Cancer treatments inducing p38 activation (in particular sunitinib, axitinib, pazopanib) stimulate the expression of VEGFC by tumor cells and promote lymphangiogenesis in clear cell renal cell carcinomas.

### Materials and Methods

### Reagents and antibodies

Sunitinib, axitinib, imatinib, everolimus, pazopanib, regorafenib, sorafenib, SB203580 and PD184352 were purchased from Selleckchem (Houston, USA). Anti-HSP90 and anti-HSP60 antibodies were purchased from Santa Cruz Biotechnology (Santa Cruz, CA, USA). Anti-p38 (ref.: 9212), anti-phospho-p38 (ref.: 4631), anti-ERK (ref.: 4695), anti-phospho-ERK antibodies were from Cell Signaling Technology (Beverly, MA, USA). TTP and HuR antibodies are home-made and were generated as previously described (Essafi-Benkhadir*et al,* 2007). DAPI, DMSO and 5,6-Dichlorobenzimidazole 1-β-D-ribofuranoside (DRB) were purchased from Sigma-Aldrich (France).

### Cell culture

RCC4 (R4), ACHN (A), Caki-2 (C2), 786-0 (786) and A-498 (498) RCC cell lines, human embryonic kidney (HEK293), RAW 264.7 (RAW) macrophage cell line were purchased from the American Tissue Culture Collection. RCC10 (R10) were a kind gift from Dr. W.H. Kaelin (Dana-Farber Cancer Institute, Boston, MA). Primary cells were already described and cultured in a medium specific for renal cells (PromoCell, Heidelberg Germany) (Grepin *et al,* 2014a).

### Immunoblotting

Cells were lysed in buffer containing 3% SDS, 10% glycerol and 0.825 mM Na₂HPO₄. 30 to 50 µg of proteins were separated on 10% SDS-PAGE, transferred onto a PVDF membrane (Immobilon, Millipore, France) and then exposed to the appropriate antibodies. Proteins were visualized with the ECL system using horseradish peroxidase-conjugated anti-rabbit or anti-mouse secondary antibodies.

### Quantitative Real-Time PCR (qPCR) experiments

One microgram of total RNA was used for the reverse transcription, using the QuantiTect Reverse Transcription kit (QIAGEN, Hilden, Germany), with blend of oligo (dT) and random primers to prime first-strand synthesis. SYBR master mix plus (Eurogentec, Liege, Belgium) was used for qPCR. The mRNA level was normalized to 36B4 mRNA. For oligo sequences also see supplemental materials.

### Tumor xenograft experiment

*Ectopic model of RCC* (Renal Cell Carcinoma): Five million 786-Ocells were injected subcutaneously into the flank of 5-week-old nude (nu/nu) female mice (Janvier, France). The tumor volume was determined with a caliper (v = L*1²*0.5). When the tumor reached 100 mm³, mice were treated five a week for 4 weeks, by gavage with placebo (dextrose water vehicle) or sunitinib (40 mg/kg). This study was carried out in strict accordance with the recommendations in the Guide for the Care and Use of Laboratory Animals. Experiments were approved by the 'Comité national institutionnel d'ethique pour l'animal de laboratoire (CIEPAL)" (reference: NCE/2013-97). Orthotopic model of RCC: Tumor samples were obtained from previously published studies involving neoadjuvant sunitinib treatment in an ortho-surgical (orthotopic tumor cell implantation followed by surgical tumor removal) of RCC (animal protocols, approvals, cell origins, and results have been previously (Ebos *et al,* 2014)). Briefly, human kidney SN12⁻PM6^{LUC+} cells (2 × 10⁶), were implanted into the kidney (subcapsular space) of 6- to 8-week-old female CB-17 SCID and treated for 14 days with sunitinib (60mg/kg/day) prior to nephrectomy.

### Immunofluorescence

Tumor sections were handled as previously described (Essafi-Benkhadir *et al,* 2007; Grepin*et al,* 2014b). Sections were incubated with DAPI, anti-mouse LYVE1 polyclonal (Ab 14817, Abcam, MA, USA) or monoclonal anti-α-smooth muscle actin (α-SMA, A2547, Sigma, France), and rats monoclonal anti-mouse CD31 (clone MEC 13.3, BD Pharmingen, NJ, USA) antibodies.

### Immunohistochemistry

Samples were collected with the approval of the Local Ethics committee. Sections from blocks of formol-fixed and paraffin-embedded tissue were examined for immunostaining for podoplanin, CD31, p-p38, αSMA and LYVE1. After deparaffinization, hydration and heat-induced antigen retrieval, the tissue sections were incubated for 20 minutes at room temperature with monoclonal anti-podoplanin, anti p-p38, anti αSMA and anti LYVE1 antibodies diluted at 1:100. Biotinylated secondary antibody [DAKO, SM802 (EnVision™ FLEX /HRP ready-to-use)] was applied and binding was detected with the substrate diaminobenzidine against a hematoxylin counterstain.

### Measurement of cytokines

After stimulation, cells supernatant was recovered for VEGFC measurement using the Human DuoSet ELISA kit (R&D Systems, MN, USA).

### Luciferase assays

Transient transfections were performed using 2 µl of lipofectamine (GIBCO BRL) and 0.5 µg of total plasmid DNA-renilla luciferase in a 500 µl final volume. The firefly control plasmid was co-transfected with the test plasmids to control for the transfection efficiency. 24 hours after transfection, cell lysates were tested for renilla and firefly luciferase. All transfections were repeated four times using different plasmid preparations. LightSwitch™ Promoter Reporter VEGFC (S710378) and LightSwitch™ 3'UTR reporter VEGFC (S803537) were purchased from Active motif (CA, USA). The short and long forms of the VEGFC promoter are a kind gift of Dr. Heide L Ford and Kari Alitalo (Wang *et al,* 2012).

### Fluorescence assays

### ARE reporter constructs and reporter activity:

RPS30 promoter-linked EGFP reporter expression vectors containing the 3'UTR with VEGFC ARE (5'-GATTTCTTTAAAAGAATGACTATATAATTTATTTCC-3') was constructed by inserting annealed synthetic complementary oligonucleotides with BamHI and XbaI overhangs into the same sites of the stable control bovine growth hormone (BGH) 3' UTR of the plasmid. The mutant ARE form was similarly constructed (5'-GATTTCTTTAAAAGAATGACTATATAATCTATTTCC-3') in which ATTTA was mutated to ATCTA (al-Haj*et al,* 2009).

### Functional response of the VEGFC ARE:

Tetracycline inducible (Tet-On) TTP expressing constructs were used as previously described (al-Haj *et al,* 2009). HEK293 Tet-On Advanced cells (Clontech, Mountain View, CA) were transfected with 50 ng of either the wild-type or mutant VEGFC 3'UTR reporters along with a normalization control represented by red fluorescent protein expression plasmid, and 10 ng of the TetO-TTP constructs. Transfections were performed using Lipofectinamine 2000 (Invitrogen) according to the manufacturer's instructions. Doxycycline (0.25 ug/ml) was added to the transfected cells for 16 hr and fluorescence was acquired by imaging and quantified by the Proxcell imaging segmentation and quantification software.

### RNA-immunoprecipitation

HEK-293 cells were transfected overnight with 2 µg vector expressing HA-tagged TTP or myc-tagged HuR. Cells were lysed in RNA IP buffer [100 mM KCl, 5 mM MgCl₂, 10 mM HEPES (pH 7.0), 0.5% NP40], freshly supplemented before use with 1 mM DTT, 5 µl/ml units RNase Out (Invitrogen) and protease inhibitor cocktail 1X (Roche). The lysate was centrifuged for 10 min at 12.000 rpm, and the supernatant was transferred to new tubes with either monoclonal anti-myc antibody or monoclonal anti-HA antibody (coupled with Protein G-sepharose beads). The beads were washed with RNA IP buffer. Aliquots were collected for immunoblotting and the remaining beads were subjected to total RNA extraction using TRI reagent (Sigma), followed by chloroform and isopropanol precipitation. Pre-swollen protein-G agarose beads (GE Healthcare) were prepared by washing in PBS buffer and incubating with the antibody, followed by PBS washing. For HA-tagged TTP lysates, anti-myc-coupled beads were used as a negative control. For myc-tagged HuR lysates, anti-HA-coupled beads were used as a negative control. cDNA was synthesized from 500 ng RNA using SuperScript II Reverse Transcriptase (Invitrogen). qPCR was performed in multiplex reaction using the C1000 thermal cycler (Bio-Rad, Hercules, CA). FAM-labeled TaqMan probes (Metabion) for human VEGF-A (Forward primer: 5'-AGAAGGAGGAGGGCAGAATC-3', Reverse primer: 5'-TCTCGATTGGATGGCAGTAG-3', and Taqman probe: 5'-Fam-CATCCAT GAACTTCACCACTTCGTGA-BHQ-1-3' and for human VEGF-C (Forward primer: 5'-GGATGCTGGAGATGACTCAA-3', Reverse primer: 5'-TTCATCCAGCTCCTTGTTTG-3' and Taqman probe: 5'-Fam-TCCACAGATGTCATGGAATCCATCTG-BHQ-1-3' were used. VIC-labeled Ribosomal Protein (PO) probe was multiplexed with FAM-labeled probes as the endogenous control to normalize for the levels of the genes of interest.

### 5,6-Dichlorobenzimidazole riboside (DRB) pulse chase experiments

25 µg/ml of DRB was added to the cells and RNAs were prepared from 0 to 4 hr thereafter. The level of VEGFC was determined by qPCR and was normalized to 36B4 mRNA. The relative amounts of VEGFC mRNA at time 0 before DRB addition were set to 100%.

### siRNA assay

siRNA transfection was performed using Lipofectamine RNAiMAX (Invitrogen). Cells were transfected with either 50 nM of si-HuR (Ambion, 4390824, s4610) or si-Control (Ambion, 4390843). After 48 hr, cells were stimulated with 5 µM of sunitinib or 5 µM of sorafenib. Two days later, qPCR was performed, as described above.

### Gene expression microarray analysis

Normalized RNA sequencing (RNA-Seq) data produced by The Cancer Genome Atlas (TCGA) were downloaded from cBiopotal (www.cbioportal.org, TCGA Provisional; RNA-Seq V2). Data were available for 503 of the 536 RCC tumor samples TCGA subjected to mRNA expression profiling. The subtype classifications were obtained through cBioPortal for Cancer Genomics and the 33 samples lacking classifications were discarded. The non-metastatic group contained 424 patients and the metastatic group contained 79 patients. The results published here are in whole or in part based upon data generated by the TCGA Research Network: http://cancergenome.nih.gov/ (Cerami*et al,* 2012; Gao*et al,* 2013). The Kaplan-Meier method was used to produce overall survival curves. The VEGFC z-score cut-off point for the overall survival was determined with the spline analysis. The effect of VEGFC and its odds-ratio was estimated using a Cox model adjusted to the expression of other genes and important patient characteristics.

### Patients and association studies

This was a retrospective study with all patients (312) consulted for a renal mass between 2008 and 2015 in Centre Antoine Lacassagne (France, Nice). Of these 312 patients, 87 only had been analyzed (cause of elimination of analyzable patients; no follow-up, renal metastasis from another cancer, RCC treated by surgery without metastasis, patients without progression, etc.). The 87 patients had metastatic RCC treated in the first line with therapies including INF+/-BVZ, sunitinib, temsirolimus. Only the patients that relapsed and patients without lymph node metastases before the treatment were included to test for the presence of lymph node metastases and new sites of metastasis on treatment (20 patients treated with sunitinib and 11 patients treated with other drugs (essentially with INF+/- BVZ)). Lymph node metastases and new sites of metastasis are two independent factors in comparison to age, gender and metastatic stage at diagnostic (M0 or M1). Neo-adjuvant patient samples were obtained from Nice, Bordeaux and Monaco Hospitals. Patients were treated for at least two months before surgery (Supplementary Table S2).

### Statistical analysis

### For in vitro and in vivo analysis

All data are expressed as the mean ± the standard error (SEM). Statistical significance and p values were determined by the two-tailed Student's *t*-test. One-way ANOVA was used for statistical comparisons. Data were analyzed with Prism 5.0b (GraphPad Software) by one-way ANOVA with Bonferroni post hoc.

### For patient analysis

All categorical data were described using frequencies and percentages. Quantitative data were presented using median and range or mean and standard deviation. Censored data were described using Kaplan-Meier estimation median survival and 95% confidence interval (CI). Statistical analyses were two sided and were considered to be significant if *p-value* ≤ 0.05 using R 3.2.2.
*Univariate analysis:* Statistical comparisons were made using χ² test or Fisher's exact test for categorical data, t-test or Wilcoxon test for quantitative data and log-rank test for censored data. Smoothing spline curves were used to predict death risk versus VEGFC mRNA expression.
*Multivariate analysis:* Multivariate analysis was carried out by creating a Cox model. Choice of the final model was made performing backward stepwise model selection. All variables associated with *p*-*value* ≤0.1 on univariate analysis were included in the model.

### Results

### Cancer treatments inducing p38 activation (in particular sunitinib, axitinib and pazopanib) stimulate VEGFC expression

In order to examine the relationship between tumor growth potential and VEGFC production, inventors used established and primary patient RCC cell lines that they developed in collaboration with the surgery department of the Nice Hospital (Grepin*et al,* 2014a) and stratified by aggressiveness. This was determined by 1) the ability to form tumors in mice (for established cell lines) and 2) the time of overall survival of patients (for primary tumor cells). They observed that increases in aggressiveness corresponded to higher levels of VEGFC mRNA (Fig. 1A) and VEGFC production (Fig. 1B). The reported intra-tumor concentrations of sunitinib in mice and patients were 5.5-13 µM (Gotink*et al,* 2014; Gotink*et al,* 2011). According to these results, they specifically used this range of concentrations in their experiments. Sunitinib induced an increase in the VEGFC mRNA level in six RCC cell lines (Fig. 1C) and in four primary tumor cells (Fig. 1D), while it did not change VEGFC mRNA in normal primary renal cells derived from three independent patients (Fig. 1E). Sunitinib increased VEGFC protein in the conditioned medium in the six RCC cell lines (Fig. 1F) and in the four independent primary cells (Fig. 1G) while it had no effect on normal cells (Fig. 1H). Inventors' results show sunitinib-induced effects are transient as VEGFC mRNA amounts return to their basal levels following treatment cessation (Figure 8A). RCC cells do not express VEGFR (qPCR ΔCt >35) but highly express CSF1R (ΔCt 29), PDGFR (ΔCt 22) and c-Kit (ΔCt 25). Hence, aberrant expression of these receptors on tumor cells that was already reported (Menke *et al,* 2012; Schiefer *et al,* 2015; Shim*et al,* 2015) may explain the effect of sunitinib. Moreover, they examined imatinib, a well-known inhibitor of c-Kit and PDGFR (like sunitinib). Inventors showed that imatinib stimulate VEGFC expression (Figure 8B). Other VEGFR TKIs (i.e., axitinib, pazopanib) were also found to increase VEGFC mRNA in 786-Ocells while bevacizumab (BVZ)/interferon and the mTOR inhibitor everolimus did not (Figure 8C).Inventors discovered that axitinib, pazopanib and imatinib activate p38. In cells adapted to a high concentration of sunitinib (10 µmol/L) (Giuliano*et al,* 2015) (Figure 9 A and B), basal VEGFC mRNA and protein amounts were increased compared to naive cells (Figure 9C and D). These results suggest a general mechanism by which drugs that directly or indirectly target angiogenesis induce VEGFC expression by tumor cells.

### Sunitinib, axitinib and pazopanib in particular stimulate VEGFC promoter activity

Sunitinib, axitinib or pazopanib-mediated induction of VEGFC mRNA suggested stimulation of transcription, stabilization of mRNA or a combination of both mechanisms. Hence, inventors first investigated the activity of the VEGFC promoter after such treatment. The treatment stimulated the activity of the VEGFC promoter in RCC cell lines (Fig. 2A) and in primary tumor cells (Fig. 2B). The transcription factor *sine oculis 1* (SIX1) participates in VEGFC transcription (Wang *et al,* 2012). Inventors generated two reporter constructs, one with a VEGFC promoter containing SIX1 binding sites (long form) and one deleted of the SIX1 consensus site (short form, Fig. 2C). The long form was stimulated by the treatment, while the short one was not, in four independent cell lines (Fig. 2D) and two primary tumor cells (Fig. 2E). VEGFC promoter activity was also higher in treatment-resistant cells (Figure 9F) resistant cells generated previously by chronic exposure to the drug (Giuliano *et al,* 2015). These results indicate transcriptional-dependent induction of VEGFC expression by sunitinib, axitinib or pazopanib-treatments.

### Sunitinib, axitinib and pazopanib induce a 3'UTR-dependent increases in the VEGFC mRNA half-life

A second mechanism that may explain treatment-induced VEGFC mRNA increases is the stabilization of VEGFC mRNA. Indeed, sunitinib, axitinib and pazopanib increased VEGFC mRNA half-life 6-fold in RCC cell lines (4.4 +/- 2.8 hr *vs* 26.5 +/- 12.9 hr) (Fig. 3A illustrating data obtained with sunitinib) and by 4-fold in primary cells (4.1 +/-3.9 hr *vs* 16.7 +/- 6.3 hr)(Fig. 3B illustrating data obtained with sunitinib). A reporter gene in which the VEGFC-3'UTR was inserted downstream of the luciferase gene (Fig. 3C) was also induced by sunitinib in two cell lines (Fig. 3D), two primary tumor cells (Fig. 3E) and in sunitinib-resistant cells (Figure 9G). These results demonstrate that such treatments enhance VEGFC expression through the stabilization of its mRNA and *via* its 3'UTR. As for VEGFA, the VEGFC mRNA 3'UTR contains an adenylate and uridylate-rich element (ARE), a binding site for HuR and TTP. Inventors used a wild-type or a VEGFC 3'UTR mutated for the ARE site coupled to the EGFP reporter gene (Fig. 3F). Expression of TTP, using a doxycycline-regulated construct, decreased the level of fluorescence only when the ARE site was present (Fig. 3G). These results indicate that TTP decreases VEGFC mRNA half-life by binding to its ARE in the 3'UTR.

### TTP and HuR bind VEGFC mRNA and modulate its half-life

Using RNA immunoprecipitation with antibodies to tagged-HuR and tagged-TTP, we found that HuR and TTP bound directly to VEGFC mRNA (Fig. 4A and B). TNFα and VEGFA mRNA binding served as positive controls and GFP and GAPDH mRNA as negative controls (Fig. 4A and B and Figure 11). The balance between TTP and HuR activity determined the relative level of the target mRNA **half-life** (Fig. 4C). The phosphorylation of HuR stimulated its ability to stabilize a given mRNA, while phosphorylation of TTP played an opposite role (Tiedje*et al,* 2012). The active, high-migrating form of TTP is detectable in RAW cells stimulated with LPS, which served as a positive control. Sunitinib, axitinib and pazopanib stimulated the expression of low-migrating inactive form of TTP and the active form of HuR (Fig. 4D illustrating data obtained with sunitinib). Overexpression of TTP decreased VEGFC 3`UTR reporter activity while overexpression of HuR increased it (Fig. 4E illustrating data obtained with sunitinib). Moreover, overexpression of TTP inhibited the treatment-dependent increase in the VEGFC 3`UTR reporter activity while overexpression of a mutated form of TTP that is poorly translated (TTPvar (Griseri*et al,* 2011)) did not (Fig. 4F). These results suggest that a regulated balance of active TTP and HuR plays a key role in the regulation of the VEGFC mRNA half-life induced by such a treatment.

### p38 and HuR are required in particular for sunitinib, axitinib and pazopanib-dependent increases in VEGFC expression

ERK and p38 pathways are critical for the modulation of the TTP and HuR activity (Sandler & Stoecklin, 2008). Sunitinib, axitinib and pazopanib induced rapid activation of the ERK and p38 pathways determined by the presence of their phosphorylated forms (p-ERK and p-p38). Activation of ERK and p38 correlated with an increase in HuR amounts and to its cytoplasmic translocation (active forms) in established RCC cell lines (Fig. 5A and Figure 12A) and in primary tumor cells (Figure 12B). Inhibition of ERK (PD184352) did not modify the treatment-dependent increase of the VEGFC mRNA, while inhibition of p38 (SB203580) strongly reduced it in an established cell line (Fig. 5B and Figure 12C) and in primary tumor cells (Figure 12D). p-p38 basal activity increased in treatment resistant cells and was correlated with enhanced VEGFC expression (Figure 9C-E). Inhibition of p38 reduced the treatment-dependent increase in the VEGFC mRNA **half-life** and the VEGFC 3'UTR reporter gene activity in an established RCC cell line (Fig. 5C and D) and in primary tumor cells (Figure 12E). Moreover, SB203580 inhibited the treatment-dependent induction of HuR (Fig. 5E). These results confirmed that VEGFC up-regulation of the mRNA **half-life** is dependent on HuR induction *via* stimulation of the p38 pathway. Hence, down-regulation of HuR with siRNA inhibited the treatment-dependent increase of VEGFC mRNA in a RCC cell line (Fig. 5F and G) and in primary tumor cells (Figure 12F and G). These results confirmed that sunitinib, axitinib and pazopanib induce a cascade of events starting from early activation of p38 to activation of HuR that finally results in induction of VEGFC mRNA expression.

### Sunitinib, axitinib, pazopanib and sorafenib in particular induce lymphangiogenesis in vivo

To correlate the treatment-dependent induction of VEFC expression to lymphangiogenesis, inventors evaluated the presence of lymphatic vessels in experimental RCC tumors obtained by subcutaneous injection of 786-Ocells in nude mice. Mice were treated with sunitinib, axitinib and pazopanib after the tumors reached 100 mm³ for approximately one month before analysis. Treatment stimulated lymphangiogenesis was confirmed by the presence of LYVE1-positive lymphatic endothelial cells (LEC) in the core of the implanted tumors after treatment while no staining was observed in the core of control tumors (Fig. 6A and B illustrating data obtained with sunitinib). p-p38 labeling was also increased in tumors of treatment-treated mice which confirmed *in vitro* observations (Fig. 6C and D illustrating data obtained with sunitinib). The levels of human (produced by xenotransplanted tumor cells) and mouse (stromal cells) VEGFC and HuR mRNA were increased only in the tumors of treated mice (Fig. 6E illustrating data obtained with sunitinib). Treatment also induced the expression of genes involved in lymphangiogenesis including *vegfr3, nrp2*, *prox1* by cells of the microenvironment (mouse) (Fig. 6E). Increased expression of pro-angiogenic genes upon treatment (*vegfa, vegfr1*, *nip1 and α-sma*) was observed (Figure 13A) but blood vessels maturation attested by coverage of endothelial cells (CD31 labeling) by pericytes (α-SMA labeling) was equivalent in control or treated mice tumors (Figure 13B). Together, these results suggest that treatment did not alter the vascular network function but induced lymphatic network development - potentially increasing tumor metastatic potential. To examine this, inventors performed a retrospective analysis of lymphatic marker expression in human RCC cells (SN12-PM6^{LUC+}) implanted orthotopically in SCID mice, neoadjuvantly treated, and then surgically resected as described by Ebos et al. (Ebos*et al,* 2014). In these studies, neoadjuvant treatment was found to have no benefit in reducing primary tumor growth but, following surgery and treatment withdrawal, increased metastasis and reduced overall survival. Inventors' results show VEGFC level increases in excised tumors from sunitinib, axitinib and pazopanib-treated mice (Fig. 6F illustrating data obtained with sunitinib) and a majority of mice that were treated in a neo-adjuvant setting developed metastasis as previously shown (Ebos *et al,* 2014). Strikingly, high VEGFC levels in tumors from treated mice were correlated with shorter survival (Fig. 6G). This observation is consistent with the development of a lymphatic network shown by the increases in PROX1 levels (Fig. 6H) and the presence of LYVE1 positive lymphatic vessels (35% in control mice vs 70% in treated mice, Fig. 6I and J). Together, these results indicate a strong correlation between sunitinib, axitinib and pazopanib treatment and a VEGFC/lymphatic vessel-dependent which, in turn, impact metastatic progression and reduce survival.

### Sunitinib, axitinib and pazopanib in particular induce lymphangiogenesis and metastasis to lymph nodes in RCC patients

There are several benefits to neoadjuvant treatment including i) the downsizing of renal tumors to facilitate surgery or ablative approaches (which can preserve renal function), ii) to assess patient sensitivity to treatment (if recurrence occurs), and iii) to prevent metastatic spread (thereby improving post-surgical survival) (Bex*et al,* 2012). However, the benefits of neoadjuvant antiangiogenic treatments have yet to be validated clinically (Bex *et al,* 2012), thus examination of patient materials is rarely performed. However, inventors obtained 13 patient samples (from a total of 3000) that were treated with antiangiogenic therapy in a neoadjuvant setting in different French hospitals (Table 1).

| **GROUP** | **CT** | **Sunitinib neoadjuvant** | **Axitinib neoadjuvant** |
|---|---|---|---|
| Number of patients | 20 | 10 | 3 |
| ccRCC | 20 (100%) | 10 (100%) | 3 (100%) |
| Sex | | | |
| - Woman | 5 (25%) | 2 (20%) | 0 (0%) |
| - Man | 15 (75%) | 8 (80%) | 3 (100%) |

| **AT DIAGNOSIS** | | | |
|---|---|---|---|
| Age | 57 (42-86) | 55 (32-79) | 56 (53-58) |
| Metastatic status | | | |
| - M0 | 10 (50%) | 2 (20%) | 3 (100%) |
| - M1 | 10(50%) | 8(80%) | 0 (0%) |
| Lymph node status | | | |
| - N0 | 15 (75%) | 6 (60%) | 3 (100%) |
| - N1 | 5 (25%) | 4 (40%) | 0 (0%) |
| Number of metastasis | | | |
| -0 | 10(50%) | 2 (20%) | 3 (100%) |
| -1 | 8 (40%) | 5 (50%) | 0 (0%) |
| -2 | 2(10%) | 3 (30%) | 0 (0%) |
| Location of metastases | | | |
| - lungs | 6 (50%) | 6 (55%) | |
| - bone | 3 (25%) | 2 (18%) | NA |
| - local | 2(17%) | 2(18%) | |
| - liver | 1 (8%) | 1 (9%) | |

| **TREATMENT (NEOADJUVANT)** | | | |
|---|---|---|---|
| Duration of treatment (months) | NA | 4.4 (2-9) | 4.7(2-6) |

Treated tumor samples were compared to tumors of untreated patients for the presence of lymphatic vessels. While lymphatic vessels were detected in four of twenty tumors (20 %) from untreated patients, inventors found lymphatic vessels in nine of thirteen tumors (69.2 %, p = 0.005) from patients treated in a neoadjuvant setting (Fig. 7A and B). The presence of lymphatic vessels correlated with an increase in VEGFR3, NRP2, PROX1, LYVE1 and HuR mRNA levels (Fig. 7C). VEGFC levels (Fig 7C) and p-p38 were not modified which inventors speculate to be due to sunitinib treatment was stopped for more than one month before surgical resection to allow wound-healing processes. This result was consistent with the transient effect of sunitinib of VEGFC expression that they observed *in vitro* (Figure 8A). Sunitinib in a neoadjuvant setting did not modify the vascular network (CD31 and a-SMA labeling, Figure 13 S and D). They next compared the presence of lymph node metastasis in non-responder patients treated with sunitinib (20 patients out of 87 with metastatic RCC (see Materials and Methods)) or other therapeutic options including interferon alpha, BVZ and a mTOR inhibitor (temsirolimus, 11 patients out of 87, Table 2).

| **GROUP** | **SUNITINIB** | **OTHER** | *p* value |
|---|---|---|---|
| Number of patients | 20 | 11 | NA |
| ccRCC | 20 (100%) | 11 (100%) | NA |
| Sex | | | |
| - Woman | 5 (25%) | 4 (36%) | 0.68 |
| - Man | 15 (75%) | 7 (64%) | |

| **AT DIAGNOSIS** | | | |
|---|---|---|---|
| Aqe | 59 (33-78) | 58 (27-78) | 0.55 |
| Metastatic status | | | |
| - M0 | 7 (35%) | 6 (54%) | 0.15 |
| - M1 | 13 (65%) | 5 (46%) | |
| Lymph node status (N0) | 20 (100%) | 11 (100%) | NA |
| Number of metastasis | | | |
| - 1 | 10 (50%) | 6 (55%) | 0.12 |
| - 2 | 9 (45%) | 2 (18%) | |
| - 3 and more | 1 (5%) | 3 (27%) | |
| Location of metastases | | | |
| - lungs | 16 (80%) | 6 (55%) | |
| - bone | 6 (30%) | 3 (27%) | 0.029 |
| - liver | 2 (18%) | 4 (36%) | |
| - brain | 0 (0%) | 4 (36%) | |
| - local | 5 (25%) | 1 (9%) | |

| **TREATMENT** | | | |
|---|---|---|---|
| Duration of treatment | 8 (2.5-14.4) | 7 (0.7-22) | 0.55 |
| Time of progression free survival (month) | 12.5 | 5.6 | 0.08 |
| Time of overall survival (month) | 23 | 42.2 | 0.012 |

| **AFTER TREATMENT** | | | |
|---|---|---|---|
| Metastatic status (M1) | 20 (100%) | 11 (100%) | NA |
| Lymph node status | | | |
| - N0 | 14 (70%) | 11 (100%) | 0.066 |
| - N1 | 6 (30%) | 0 (0%) | |
| Number of metastases have progressed (except lymph node) | | | |
| - 1 | 9 (45%) | 10 (91%) | 0.034 |
| - 2 | 6 (55%) | 1 (9%) | |
| - 3 and more | 5 (25%) | 0 (0%) | |
| Location of metastases have progressed (except lymph node) | | | |
| - lungs | 13 (65%) | 6 (55%) | |
| - bone | 5 (25%) | 1 (9%) | 0.87 |
| - liver | 7 (35%) | 1 (9%) | |
| - brain | 6 (30%) | 3 (27%) | |
| - local | 4 (20%) | 1 (9%) | |
| Number of news sites metastases (except lymph node) | | | |
| - 0 | 6 (30%) | 7 (64%) | |
| - 1 | 7 (35%) | 4 (37%) | 0.15 |
| - 2 | 5 (25%) | 0 (0%) | |
| - 3 and more | 2 (10%) | 0 (0%) | |
| Location of news sites metastases (except lymph node) | | | |
| - lungs | 3 (15%) | 0(0%) | |
| - bone | 4 (20%) | 0 (0%) | 0.93 |
| - liver | 4 (20%) | 1 (9%) | |
| - brain | 6 (30%) | 1 (9%) | |
| - local | 5 (25%) | 2 (18%) | |

35% of patients treated with sunitinib developed lymph node metastasis and new metastatic sites, whereas patients treated with other therapeutic options did not (*p*=0.033) (Fig. 7D). As expected, patients with invaded lymph nodes (N1) and new metastatic sites had a shorter overall survival (*p*=0.012) (Fig. 7D).

This result further reinforces inventors' conclusion that sunitinib, axitinib and pazopanib stimulates the development of a lymphatic network. Finally, cBioPortal analysis showed that high amounts of VEGFC correlated with decreased overall survival (*p*=0.0026, Figure 14A) and an increased proportion of metastatic patients ((M1 patients) *p* = 0.019, Figure 14B). The level of VEGFC, the tumor stage, the metastatic status (M1) and the lymph node invasion (N1) were analyzed in a multivariate Cox regression model on overall survival. VEGFC expression was identified as an independent prognostic parameter for overall survival (p = 0.000253, Figure 14C).

### Conclusion

Resistance to anti-angiogenic treatments are classified into: *i*) intrinsic resistance; tumors fail to respond from the outset of treatment, through sequestration of sunitinib in lysosomes (Giuliano *et al,* 2015), and *ii*) acquired resistance; compensatory pathways (Sennino*et al,* 2012) including an increase in lymphangiogenesis, as herein shown by inventors. Eighty percent of metastases of solid cancers are estimated to disseminate through the lymphatic system, while 20% of metastases may occur through the blood vasculature or by direct seeding (Alitalo & Detmar, 2012). Lymph node metastasis is the first sign of tumor progression in the majority of epithelial malignancies (Alitalo & Detmar, 2012) (invasion of sentinel lymph nodes in breast cancers is directly linked to prognosis). An increased density of peri- and intra-tumor lymphatic vessels, as inventors observed in tumors from mice treated with a cancer treatment activating p38, indicates activation of lymphangiogenesis. A significant correlation has been observed between lymphatic vessel density and lymph node and organ metastasis. High expression levels of the lymphangiogenic factor VEGFC correlates with lymph node metastasis in numerous tumor types. Overexpression of VEGFC or VEGFD in mouse models increases the lymphatic vessel density and diameter of lymph nodes and organ metastasis (Alitalo & Detmar, 2012). Intra-tumor lymphatic vessels and increased metastasis had been observed in VEGFC-overexpressing tumors implanted in mice (Alitalo & Detmar, 2012). Furthermore, lymphatic invasion by RCC cells was the only independent risk factor for lymph node metastasis (Ishikawa*et al,* 2007). Whereas sunitinib increased VEGFC transcription and mRNA stabilization, it did not increase VEGFD expression in inventors' RCC model systems. Strikingly, VEGFD mRNA 3'UTR does not contain ARE. Cancer treatments activating p38 increased VEGFC expression only in tumor cells and not in normal cells (kidney cells and LEC), suggesting specific genetic particularities that mediate tumor cell adaptation to a toxic drug. Surprisingly, Regorafenib, another TKI with the same targets as sunitinib did not stimulate VEGFC expression. Inventors discovered that unlike sunitinib, regorafenib inhibits p38 activity, which reinforced the specific role of p38 in the sunitinib, axitinib or pazopanib-dependent increase of VEGFC expression. Regulation of VEGFC expression has been poorly addressed (Enholm*et al,* 1997). *Vegfc* gene transcription depends on the transcription factor SIX1, which is overexpressed in metastatic breast cancers (Wang *et al,* 2012). The critical role for SIX1 in lymphatic dissemination of breast cancer cells provides a direct mechanistic explanation linking VEGFC expression, lymphangiogenesis and metastasis (Wang *et al,* 2012). Sunitinib did not stimulate the VEGFC promoter deleted of the domain containing SIX1 binding sites suggesting that it may play a role. However, the deleted domain also contains binding sites for NF-KappaB, GATA-2 and 3, Erg-1 and p53 transcription factors. Hence, further experiments are needed to determine the transcription factors implicated in the sunitinib-dependent stimulation of *vegfc* gene transcription. Analysis of available online databases (TGCA) with cBioPortal (http://www.cbioportal.org) showed that overexpression of SIX1 does not correlate with short progression-free or overall survival for patients with metastatic RCC unlike for breast cancers patients whereas VEGFC is a factor of poor prognosis suggesting that SIX1 is not the major driver of *vegfc* gene transcription in these tumors. VEGFC expression is regulated at the level of its mRNA half-life by a subtle balance between HuR and TTP. By increasing the half-life of a specific mRNA, HuR enhances the levels of proteins that promote cell proliferation, increase cell survival and local angiogenesis, help the cancer cells to evade immune recognition and facilitate cancer cell invasion and metastasis (Abdelmohsen & Gorospe, 2010). High levels of cytoplasmic HuR have been found in oral, colorectal, gastric, lung, breast, ovarian, renal, skin carcinomas, and mesothelioma. Stromal cells and adjacent non-tumor tissues do not show cytoplasmic expression of HuR (Govindaraju & Lee, 2013). Cytoplasmic HuR expression is associated with reduced RCC survival (Ronkainen*et al,* 2011) and down-regulation of HuR inhibits cell proliferation and induces apoptosis of RCC cells (Danilin*et al,* 2010). Cytoplasmic expression of HuR is associated with lymph node metastasis and advanced disease in non-small cell lung, colon and upper urinary tract urothelial carcinomas. The cytoplasmic levels of HuR are increased in tumors with lymphatic/vascular invasion compared to tumors without vessel invasion in cervical, colon, and *in situ* breast ductal carcinomas (Govindaraju & Lee, 2013). These results are consistent with sunitinib, axitinib or pazopanib-dependent activation of HuR, increased VEGFC mRNA half-life and lymphangiogenesis. Sunitinib, axitinib and pazopanib were shown to diminish the postsurgical benefits of neoadjuvant sunitinib, axitinib or pazopanib treatment in mice, including the promotion of metastasis (and decrease in survival) of select RCC models. Initial observations suggested that sunitinib modified the localization of the metastases and could increase incidence in the lymph nodes, spleen and stomach, two organs drained to a large extent by lymphatic vessels (Ebos *et al,* 2014). These findings are consistent with inventors results showing that sunitinib, axitinib and pazopanib increased VEGFC expression and lymphangiogenesis. Sunitinib also induced VEGFR2 and VEGFR3 expression in LEC *in vitro* and in stromal cells *in vivo*.Expression may favor the paracrine action of VEGFC overexpressed by tumor cells after sunitinib treatment on LEC and the development of a lymphatic network in the tumor. Whereas sunitinib inhibits VEGFR2 and VEGFR3, the accumulation of VEGFC may act during the intercure (four weeks of treatment interrupted two weeks to limit toxic effects). Alternatively, VEGFC may stimulate neuropilin-2 (NRP2), the co-receptor of VEGFR3, which is overexpressed on RCC cells (Cao*et al,* 2013). The importance of NRP2 during the initiation of new lymphatic vessel sprouts was detected in hypoplastic lymphatic vessels observed in *nrp2-*gene targeted mice. Among other structures, NRP2 is expressed on veins and up-regulated in tumor-associated lymphatic vessels where it binds VEGFC, VEGFA and partially processed VEGFD (Alitalo & Detmar, 2012). Moreover, blocking NRP2 function inhibits tumor cell metastasis (Caunt*et al,* 2008). NRP2 expressed on cancer cells interacts with alpha 5 integrin on endothelial cells to mediate vascular extravasation and promotion of metastasis in zebrafish and murine xenograft models of RCC and pancreatic adenocarcinoma (Cao *et al,* 2013). Moreover, NRP2 correlates with poor prognosis in patients with advanced RCC. The median overall survival was longer for patients with low levels of NRP2 (26 months) compared to patients overexpressing NRP2 (13 months) (Cetin*et al,* 2015). NRP2 mRNA is increased in tumors of mice treated with sunitinib. For this reason, co-treatment with sunitinib, axitinib or pazopanib and VEGFC blocking agents may be a good option for RCC patients to reduce the progression of the disease. This co-treatment probably results in the reduction of the dose of cancer treatment activating p38, avoiding intercure during which lymphatic vessels may develop.

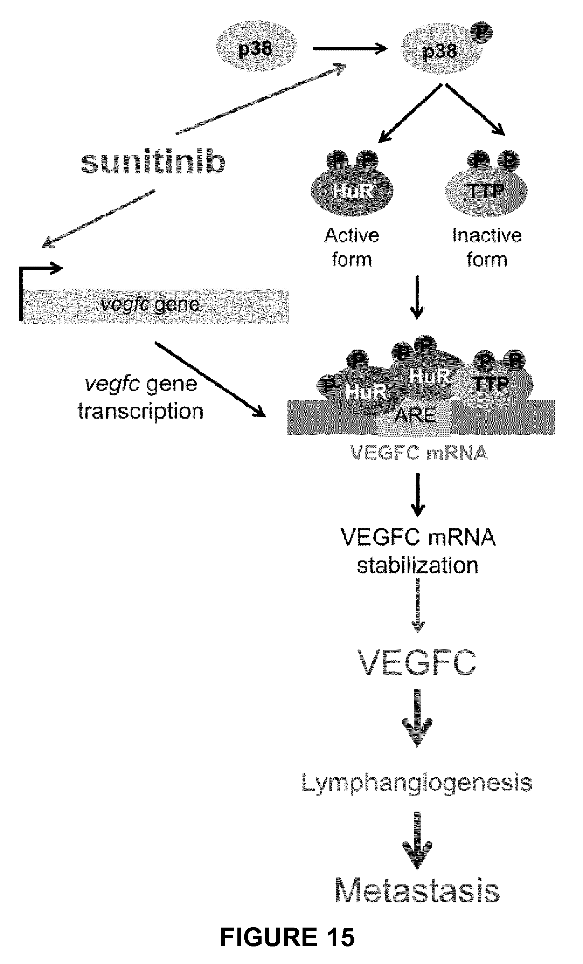

Inventors herein showed that sunitinib, axitinib and pazopanib correlated to lymphatic invasion in experimental and human tumors. A quick appraisal of these results could be that such a treatment is detrimental for patients, which is absolutely not the case (Table 2) (Blagoev *et al,* 2013; Miles *et al,* 2011). Inventors' results confirm that sunitinib, axitinib and pazopanib compared to other treatments prolonged survival but suggest a fraction of the non-responder patients had more invaded lymph nodes and new metastatic sites compared to non-responder patients treated with other therapeutic agents, a potential reason for progression on treatment. Inventors' results demonstrate that combining VEGFC inhibitors with sunitinib, axitinib or pazopanib will limit the progression of the disease. In an analyzed cohort, twenty-eight percent of patients stopped sunitinib treatment because of intolerance. New TKI like axitinib or pazopanib are better tolerated or provide a better health-related quality of life (Escudier*et al,* 2014). Moreover, they have an acidic pKa that prevents their sequestration in lysosomes, a mechanism associated with resistance to sunitinib (Giuliano *et al,* 2015). However, both inhibitors stimulated VEGFC expression (Supplementary Fig. S1). This is another argument for the combination of cancer treatment activating p38with VEGFC inhibitors, typically with an anti-VEGFc antibody. To conclude, overexpression of VEGFC represents an extrinsic mechanism of adaptation of RCC leading to treatment resistance. A recapitulated schema is shown in Figure 15. Although treatments such as sunitinib, axitinib, pazopanib and imatinib have revolutionized the care of patients, their efficacy may be improved by targeting VEGFC-dependent development of the lymphatic network, a major route of spread of tumor cells when the patients become resistant to therapy.

In addition to the induction of VEGFC mediated by sunitinib, pazopanib, axitinib and imatinib in RCC, inventors are testing the activation of p38 and induction of VEGFC by the standard treatment used for other metastatic solid cancers including platinum salts for head and neck and vulval cancers, and taxanes for melanoma, breast (triple negative) and prostate cancers. In model cell lines of these different tumors types, inventors observed p38 activation and VEGFC induction in response to the treatments. These results suggest that VEGFC increase is a general stress response associated with any therapies. The general concept combining anti-VEGFC treatment with the therapy of reference when said therapy induces p38 activation may be extended to any carcinoma, sarcoma, melanoma and pediatric tumour, in particular to any breast cancer, ovarian cancer, lung cancer (in particular NSCLC), melanoma, head and neck cancer, colon cancer and mesothelioma.

### REFERENCES

- Abdelmohsen K, Gorospe M (2010) Posttranscriptional regulation of cancer traits by HuR. Wiley Interdiscip Rev RNA 1: 214-229
- al-Haj L, Al-Ahmadi W, Al-Saif M, Demirkaya O, Khabar KS (2009) Cloning-free regulated monitoring of reporter and gene expression. BMC Mol Biol 10: 20
- Alitalo A, Detmar M (2012) Interaction of tumor cells and lymphatic vessels in cancer progression. Oncogene 31: 4499-4508
- Belum VR, Wu S, Lacouture ME (2013) Risk of hand-foot skin reaction with the novel multikinase inhibitor regorafenib: a meta-analysis. Invest New Drugs 31: 1078-1086
- Bex A, Kroon BK, de Bruijn R (2012) Is there a role for neoadjuvant targeted therapy to downsize primary tumors for organ sparing strategies in renal cell carcinoma? Int J Surg Oncol 2012: 250479
- Blagoev KB, Wilkerson J, Stein WD, Motzer RJ, Bates SE, Fojo AT (2013) Sunitinib does not accelerate tumor growth in patients with metastatic renal cell carcinoma. Cell Rep 3: 277-281
- Brennan SE, Kuwano Y, Alkharouf N, Blackshear PJ, Gorospe M, Wilson GM (2009) The mRNA-destabilizing protein tristetraprolin is suppressed in many cancers, altering tumorigenic phenotypes and patient prognosis. Cancer Res 69: 5168-5176
- Cao Y, Hoeppner LH, Bach S, E G, Guo Y, Wang E, Wu J, Cowley MJ, Chang DK, Waddell N, Grimmond SM, Biankin AV, Daly RJ, Zhang X, Mukhopadhyay D (2013) Neuropilin-2 promotes extravasation and metastasis by interacting with endothelial alpha5 integrin. Cancer Res 73: 4579-4590
- Caunt M, Mak J, Liang WC, Stawicki S, Pan Q, Tong RK, Kowalski J, Ho C, Reslan HB, Ross J, Berry L, Kasman I, Zlot C, Cheng Z, Le Couter J, Filvaroff EH, Plowman G, Peale F, French D, Carano R et al (2008) Blocking neuropilin-2 function inhibits tumor cell metastasis. Cancer Cell 13: 331-342
- Cerami E, Gao J, Dogrusoz U, Gross BE, Sumer SO, Aksoy BA, Jacobsen A, Byrne CJ, Heuer ML, Larsson E, Antipin Y, Reva B, Goldberg AP, Sander C, Schultz N (2012) The cBio cancer genomics portal: an open platform for exploring multidimensional cancer genomics data. Cancer Discov 2: 401-404
- Cetin B, Gonul, II, Buyukberber S, Afsar B, Gumusay O, Algin E, Turan N, Ozet A, Benekli M, Coskun U (2015) The impact of immunohistochemical staining with ezrin-carbonic anhydrase IX and neuropilin-2 on prognosis in patients with metastatic renal cell cancer receiving tyrosine kinase inhibitors. Tumour Biol 36: 8471-8478
- Danilin S, Sourbier C, Thomas L, Lindner V, Rothhut S, Dormoy V, Helwig JJ, Jacqmin D, Lang H, Massfelder T (2010) Role of the RNA-binding protein HuR in human renal cell carcinoma. Carcinogenesis 31: 1018-1026
- Ebos JM (2015) Prodding the Beast: Assessing the Impact of Treatment-Induced Metastasis. Cancer Res 75: 3427-3435
- Ebos JM, Lee CR, Cruz-Munoz W, Bjarnason GA, Christensen JG, Kerbel RS (2009) Accelerated metastasis after short-term treatment with a potent inhibitor of tumor angiogenesis. Cancer Cell 15: 232-239
- Ebos JM, Mastri M, Lee CR, Tracz A, Hudson JM, Attwood K, Cruz-Munoz WR, Jedeszko C, Burns P, Kerbel RS (2014) Neoadjuvant antiangiogenic therapy reveals contrasts in primary and metastatic tumor efficacy. EMBO Mol Med 6: 1561-1576
- Eisen T, Sternberg CN, Robert C, Mulders P, Pyle L, Zbinden S, Izzedine H, Escudier B (2012) Targeted therapies for renal cell carcinoma: review of adverse event management strategies. JNatl Cancer Inst 104: 93-113
- Enholm B, Paavonen K, Ristimäki A, Kumar V, Gunji Y, Klefstrom J, Kivinen L, Laiho M, Olofsson B, Joukov V, Eriksson U, Alitalo K (1997) Comparison of VEGF, VEGF-B, VEGF-C and Ang-1 mRNA regulation by serum, growth factors, oncoproteins and hypoxia. Oncogene 14: 2475-2483
- Escudier B (2010) Sunitinib for the management of advanced renal cell carcinoma. Expert Rev Anticancer Ther 10: 305-317
- Escudier B, Porta C, Bono P, Powles T, Eisen T, Sternberg CN, Gschwend JE, De Giorgi U, Parikh O, Hawkins R, Sevin E, Negrier S, Khan S, Diaz J, Redhu S, Mehmud F, Cella D (2014) Randomized, controlled, double-blind, cross-over trial assessing treatment preference for pazopanib versus sunitinib in patients with metastatic renal cell carcinoma: PISCES Study. J Clin Oncol 32: 1412-1418
- Essafi-Benkhadir K, Onesto C, Stebe E, Moroni C, Pages G (2007) Tristetraprolin inhibits Ras-dependent tumor vascularization by inducing vascular endothelial growth factor mRNA degradation. Mol Biol Cell 18: 4648-4658
- Gao J, Aksoy BA, Dogrusoz U, Dresdner G, Gross B, Sumer SO, Sun Y, Jacobsen A, Sinha R, Larsson E, Cerami E, Sander C, Schultz N (2013) Integrative analysis of complex cancer genomics and clinical profiles using the cBioPortal. Sci Signal 6: p11
- Giuliano S, Cormerais Y, Dufies M, Grepin R, Colosetti P, Belaid A, Parola J, Martin A, Lacas-Gervais S, Mazure NM, Benhida R, Auberger P, Mograbi B, Pages G (2015) Resistance to sunitinib in renal clear cell carcinoma results from sequestration in lysosomes and inhibition of the autophagic flux. Autophagy 11: 1891-1904
- Giuliano S, Pages G (2013) Mechanisms of resistance to anti-angiogenesis therapies. Biochimie 95: 1110-1119
- Gotink KJ, Broxterman HJ, Honeywell RJ, Dekker H, de Haas RR, Miles KM, Adelaiye R, Griffioen AW, Peters GJ, Pili R, Verheul HM (2014) Acquired tumor cell resistance to sunitinib causes resistance in a HT-29 human colon cancer xenograft mouse model without affecting sunitinib biodistribution or the tumor microvasculature. Oncoscience 1: 844-853
- Gotink KJ, Broxterman HJ, Labots M, de Haas RR, Dekker H, Honeywell RJ, Rudek MA, Beerepoot LV, Musters RJ, Jansen G, Griffioen AW, Assaraf YG, Pili R, Peters GJ, Verheul HM (2011) Lysosomal sequestration of sunitinib: a novel mechanism of drug resistance. Clin Cancer Res 17: 7337-7346
- Govindaraju S, Lee BS (2013) Adaptive and maladaptive expression of the mRNA regulatory protein HuR. World JBiol Chem 4: 111-118
- Grau S, Thorsteinsdottir J, von Baumgarten L, Winkler F, Tonn JC, Schichor C (2011) Bevacizumab can induce reactivity to VEGF-C and -D in human brain and tumour derived endothelial cells. J Neurooncol 104: 103-112
- Grepin R, Ambrosetti D, Marsaud A, Gastaud L, Amiel J, Pedeutour F, Pages G (2014a) The relevance of testing the efficacy of anti-angiogenesis treatments on cells derived from primary tumors: a new method for the personalized treatment of renal cell carcinoma. PLoS ONE 9: e89449
- Grepin R, Guyot M, Giuliano S, Boncompagni M, Ambrosetti D, Chamorey E, Scoazec JY, Negrier S, Simonnet H, Pages G (2014b) The CXCL7/CXCR1/2 axis is a key driver in the growth of clear cell renal cell carcinoma. Cancer Res 74: 873-883
- Griseri P, Bourcier C, Hieblot C, Essafi-Benkhadir K, Chamorey E, Touriol C, Pages G (2011) A synonymous polymorphism of the Tristetraprolin (TTP) gene, an AU-rich mRNA-binding protein, affects translation efficiency and response to Herceptin treatment in breast cancer patients. Hum Mol Genet 20: 4556-4568
- Griseri P, Pages G (2014) Regulation of the mRNA half-life in breast cancer. Word J Clin Oncol 5: 323-334
- Ishikawa Y, Aida S, Tamai S, Akasaka Y, Kiguchi H, Akishima-Fukasawa Y, Hayakawa M, Soh S, Ito K, Kimura-Matsumoto M, Ishiguro S, Nishimura C, Kamata I, Shimokawa R, Ishii T (2007) Significance of lymphatic invasion and proliferation on regional lymph node metastasis in renal cell carcinoma. Am J Clin Pathol 128: 198-207
- Levy NS, Chung S, Furneaux H, Levy AP (1998) Hypoxic stabilization of vascular endothelial growth factor mRNA by the RNA-binding protein HuR. J Biol Chem 273: 6417-6423
- Li D, Xie K, Ding G, Li J, Chen K, Li H, Qian J, Jiang C, Fang J (2014) Tumor resistance to anti-VEGF therapy through up-regulation ofVEGF-C expression. Cancer Lett 346: 45-52
- Menke J, Kriegsmann J, Schimanski CC, Schwartz MM, Schwarting A, Kelley VR (2012) Autocrine CSF-1 and CSF-1 receptor coexpression promotes renal cell carcinoma growth. Cancer Res 72: 187-200
- Miles D, Harbeck N, Escudier B, Hurwitz H, Saltz L, Van Cutsem E, Cassidy J, Mueller B, Sirzen F (2011) Disease course patterns after discontinuation of bevacizumab: pooled analysis of randomized phase III trials. J Clin Oncol 29: 83-88
- Mueller C1, Edmiston KH, Carpenter C, Gaffney E, Ryan C, Ward R, White S, Memeo L, Colarossi C, Petricoin EF 3rd, Liotta LA, Espina V. One-step preservation of phosphoproteins and tissue morphology at room temperature for diagnostic and research specimens. PloS one 2011; 6(8):e23780.
- Paez-Ribes M, Allen E, Hudock J, Takeda T, Okuyama H, Vinals F, Inoue M, Bergers G, Hanahan D, Casanovas O (2009) Antiangiogenic therapy elicits malignant progression of tumors to increased local invasion and distant metastasis. Cancer Cell 15: 220-231
- Ribatti D (2016) Tumor refractoriness to anti-VEGF therapy. Oncotaiget
- Ronkainen H, Vaarala MH, Hirvikoski P, Ristimaki A (2011) HuR expression is a marker of poor prognosis in renal cell carcinoma. Tumour Biol 32: 481-487
- Sandler H, Stoecklin G (2008) Control of mRNA decay by phosphorylation of tristetraprolin. Biochem Soc Trans 36: 491-496
- Schiefer AI, Mesteri I, Berghoff AS, Haitel A, Schmidinger M, Preusser M, Birner P (2015) Evaluation of tyrosine kinase receptors in brain metastases of clear cell renal cell carcinoma reveals cMet as a negative prognostic factor. Histopathology 67: 799-805
- Sennino B, Ishiguro-Oonuma T, Schriver BJ, Christensen JG, McDonald DM (2013) Inhibition of c-Met reduces lymphatic metastasis in RIP-Tag2 transgenic mice. Cancer-Res 73: 3692-3703
- Sennino B, Ishiguro-Oonuma T, Wei Y, Naylor RM, Williamson CW, Bhagwandin V, Tabruyn SP, You WK, Chapman HA, Christensen JG, Aftab DT, McDonald DM (2012) Suppression of tumor invasion and metastasis by concurrent inhibition of c-Met and VEGF signaling in pancreatic neuroendocrine tumors. Cancer Discov 2: 270-287
- Shim M, Song C, Park S, Choi SK, Cho YM, Kim CS, Ahn H (2015) Prognostic significance of platelet-derived growth factor receptor-beta expression in localized clear cell renal cell carcinoma. J Cancer Res Clin Oncol 141: 2213-2220
- Su JL, Yen CJ, Chen PS, Chuang SE, Hong CC, Kuo IH, Chen HY, Hung MC, Kuo ML (2007) The role of the VEGF-C/VEGFR-3 axis in cancer progression. Br J Cancer 96: 541-545
- Tiedje C, Ronkina N, Tehrani M, Dhamija S, Laass K, Holtmann H, Kotlyarov A, Gaestel M (2012) The p38/MK2-driven exchange between tristetraprolin and HuR regulates AU-rich element-dependent translation. PLoS Genet 8: e1002977
- Wang CA, Jedlicka P, Patrick AN, Micalizzi DS, Lemmer KC, Deitsch E, Casas-Selves M, Harrell JC, Ford HL (2012) SIX1 induces lymphangiogenesis and metastasis via upregulation of VEGF-C in mouse models of breast cancer. J Clin Invest 122: 1895-1906
- Wang J, Guo Y, Chu H, Guan Y, Bi J, Wang B (2013) Multiple functions of the RNA-binding protein HuR in cancer progression, treatment responses and prognosis. Int J Mol Sci 14: 10015-10041
- Wang Z, Dabrosin C, Yin X, Fuster MM, Arreola A, Rathmell WK, Generali D, Nagaraju GP, El-Rayes B, Ribatti D, Chen YC, Honoki K, Fujii H, Georgakilas AG, Nowsheen S, Amedei A, Niccolai E, Amin A, Ashraf SS, Helferich B et al (2015) Broad targeting of angiogenesis for cancer prevention and therapy. Semin Cancer Biol 35 Suppl: S224-243
- Welti J, Loges S, Dimmeler S, Carmeliet P (2013) Recent molecular discoveries in angiogenesis and antiangiogenic therapies in cancer. J Clin Invest 123: 3190-3200

## Claims

1. An anti-VEGFC antibody, or a functional fragment thereof, for use for preventing the occurrence of new metastases or the development of existing metastases in a subject exposed to or who will be exposed to a cancer treatment inducing p38 activation selected from axitinib, pazopanib, imatinib, a taxan and a platin.

2. The anti-VEGFC antibody for use according to claim 1, wherein the cancer treatment inducing p38 activation is a taxan selected from docetaxel, paclitaxel and cabazitaxel.

3. The anti-VEGFC antibody for use according to claim 1, wherein the cancer treatment inducing p38 activation is a platin selected from cisplatin, carboplatin and oxaliplatin.

4. The anti-VEGFC antibody for use according to anyone of claims 1 to 3, wherein the subject is a human being.

5. The anti-VEGFC antibody for use according to anyone of claims 1, 3 or 4, wherein the subject is exposed to or will be exposed to a neoadjuvant cancer treatment inducing p38 activation selected from axitinib, pazopanib and imatinib.

6. The anti-VEGFC antibody for use according to anyone of claims 1, 3, 4 or 5, wherein the cancer treatment inducing p38 activation is axitinib.

7. The anti-VEGFC antibody for use according to anyone of the preceding claims, wherein the cancer treatment is part of a treatment protocol comprising a step of assessing p38 activation in at least one metastasis or metastatic cell of the subject.

8. The anti-VEGFC antibody for use according to claim 7, wherein the step of assessing p38 activation occurs after the administration of the cancer treatment inducing p38 activation to the subject.

9. The anti-VEGFC antibody for use according to claim 7, wherein the step of assessing p38 activation occurs 4 to 12 months following the first administration of the cancer treatment inducing p38 activation to the subject and/or when a 5 to 20% increase of the volume of at least one metastasis has been measured since the first administration of the cancer treatment inducing p38 activation.

10. The anti-VEGFC antibody for use according to anyone of claims 1 to 9, wherein the cancer treatment inducing p38 activation and the anti-VEGFC antibody are administered sequentially, the anti-VEGFC antibody being administered after the cancer treatment inducing p38 activation, preferably when p38 activation has been detected in at least one metastasis or metastatic cell of the subject.

11. A cancer treatment inducing p38 activation selected from axitinib, pazopanib, imatinib, a taxan and a platin, for use in combination with an anti-VEGFC antibody, or a functional fragment thereof, for treating a cancer and preventing metastasis in a subject in need thereof, preferably for use sequentially, the anti-VEGFC antibody being administered after the cancer treatment inducing p38 activation.

12. A method for selecting an optimal therapeutic treatment of cancer in a subject having metastasis(es) exposed to a cancer treatment inducing p38 activation selected from axitinib, pazopanib, imatinib, a taxan and a platin, wherein the method comprises a step of assessing p38 activation *ex vivo* in at least one metastasis or metastatic cell of the subject, and a step of deciding that the cancer treatment inducing p38 activation is to be combined to the administration to the subject of an anti-VEGFC antibody, or of a functional fragment thereof, as the optimal therapeutic treatment of cancer for the subject, if an activation of p38 is detected in the at least one metastasis or metastatic cell of the subject.

13. Kit comprising (i) a cancer treatment inducing p38 activation selected from axitinib, pazopanib, imatinib, a taxan and a platin, and (ii) an anti-VEGFC antibody or a functional fragment thereof,.

14. Kit according to claim 12 for use for treating cancer and preventing the occurrence of new metastasis(es) or the development of existing metastasis(es) in a subject.

15. Kit comprising at least one anti-p38 antibody for detecting p38 activation in a metastasis or metastatic cell from a subject suffering of a cancer, and at least one secondary antibody detecting the at least one anti-p38 antibody, optionally together with an anti-VEGFC antibody or a functional fragment thereof.
